# EUROPÄISCHE PATENTSCHRIFT

(11) **EP 3 297 603 B1**
(45) Veröffentlichungstag und Bekanntmachung des Hinweises auf die Patenterteilung: **08.05.2019**
(21) Anmeldenummer: 16724629.7
(22) Anmeldetag: 20.05.2016
(51) Int. Cl.: A61K 9/00, A61K 47/10, A61K 47/14, A61K 45/06, A61K 31/164, A61K 31/19, A61K 31/351, A61K 31/4174, A61K 31/505, A61K 47/12

(54) **CINEOLHALTIGE WÄSSRIGE ZUSAMMENSETZUNG FÜR DIE NASALE APPLIKATION**
CINEOLE-CONTAINING AQUEOUS COMPOSITION FOR NASAL APPLICATION
COMPOSITION AQUEUSE CONTENANT DU CINÉOL POUR APPLICATION NASALE

(30) Priorität: 01.09.2015 DE 102015011158; 03.09.2015 DE 102015011331; 08.09.2015 DE 102015115107
(43) Veröffentlichungstag der Anmeldung: 28.03.2018
(73) Patentinhaber: Maria Clementine Martin Klosterfrau Vertriebsgesellschaft mbH, 50670 Köln (DE)
(72) Erfinder: UNKAUF, Markus, 80538 München (DE); PLOCH, Michael, 16515 Oranienburg (DE); GALAN-SOUSA, José, 50670 Köln (DE)
(74) Vertreter: Von Rohr Patentanwälte Partnerschaft mbB
(86) Internationale Anmeldenummer: PCT/EP2016/061430
(87) Internationale Veröffentlichungsnummer: WO 2017/036617

(56) Entgegenhaltungen:
- DE-A1-102013 001 151
- DE-U1-202014 105 553
- US-B1- 6 482 397

## Beschreibung

Die vorliegende Erfindung betrifft das technische (d. h. medizinisch-therapeutische) Gebiet der Behandlung von Rhinitiden.

Insbesondere betrifft die vorliegende Erfindung eine cineolhaltige Zusammensetzung, insbesondere eine cineolhaltige pharmazeutische Zusammensetzung, welche sich für die topische, insbesondere nasale, bevorzugt intranasale Applikation eignet, nämlich für die Behandlung von Rhinitiden. Des Weiteren betrifft die vorliegende Erfindung auch ein Stabilisierungsverfahren (d. h. ein Verfahren zur Stabilisierung einer cineolhaltigen Zusammensetzung nach der vorliegenden Erfindung). Die erfindungsgemäße cineolhaltige Zusammensetzung wird nachfolgend gelegentlich synonym auch als erfinderische Kombination bzw. erfinderische Wirkstoffkombination oder als erfinderisches Antirhinitismittel (Antirhinitikum) bezeichnet.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen (z. B. viralen oder bakteriellen), allergischen oder pseudoallergischen Ursprungs sein kann. Am Häufigsten tritt eine Rhinitis im Rahmen einer sogenannten Erkältung, aber auch im Fall von sogenanntem Heuschnupfen auf.

Neben einer Unterscheidung von akuter Rhinitis einerseits und chronischer Rhinitis andererseits unterscheidet man auch verschiedene Formen von Rhinitiden, so beispielsweise die folgenden Rhinitisformen: *Rhinitis acuta*, *Rhinitis atrophicans*, *Rhinitis allergica, Rhinitis hypertrophica, Rhinitis medicamentosa, Rhinitis pseudomembranacea, Rhinitis sicca*, *Rhinitis vasomotorica* und die umweltbedingte Rhinitis.

Bei der sogenannten akuten Rhinitis (*Rhinitis acuta*), d. h. dem gewöhnlichen Schnupfen, handelt es sich in der Regel um einen im Allgemeinen harmlosen Infekt der Nasenschleimhäute und damit um eine infektiöse Rhinitis, welche meist viral, insbesondere durch eine Vielzahl von Viren (insbesondere Rhinoviren und/oder Adenoviren), gelegentlich aber auch bakteriell ausgelöst werden kann. Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Bei einer allergischen Rhinitis (*Rhinitis allergica*) kann es sich insbesondere entweder um eine saisonale allergische Rhinopathie, insbesondere verursacht z. B. durch Pflanzenpollen, Konidien extramuraler Pilze etc., oder aber um eine perenniale, nicht jahreszeitlich gehäuft auftretende Rhinopathie, insbesondere verursacht durch häusliche Allergene (z. B. Hausstaubmilben, intramurale Pilze, Haustierepithelien etc.) oder durch Arbeitsplatzallergene (z. B. Mehl-, Holz-, Medikamenten-, Pflanzenstäube etc.), handeln.

Eine Rhinitis (d. h. eine Entzündung der Nasenschleimhaut), insbesondere eine akute Rhinitis, kann oftmals gleichzeitig auch mit einer Entzündung der Schleimhaut der Nasennebenhöhlen (Sinusitis) vorliegen. In einem solchen Fall spricht man auch von einer Rhinosinusitis.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter: "*Rhinitis*", "*Rhinitis allergica*", "*Rhinitis atrophicans*", "*Rhinitis hyperplastica*", "*Rhinitis pseudomembranacea*"*, "Rhinitis sicca"* und "*Rhinitis vasomotorica*"*.*

Da es also eine Vielzahl verschiedener Typen von Viren gibt, welche eine akute virale Rhinitis auslösen können, bzw. da eine Vielzahl von Ursachen für das Auftreten einer Rhinitis existieren, können Rhinitiden, insbesondere akute, aber auch allergische Rhinitiden, im Allgemeinen nicht kausal, sondern nur symptomatisch, insbesondere topisch, behandelt werden.

Zu diesem Zweck kommen in den meisten Fällen intranasal zu applizierende Zusammensetzungen zur Anwendung, welche oftmals sogenannte Sympathomimetika (synonym auch als "Abschweller", "Dekongestiva" oder dergleichen bezeichnet), vorzugweise alpha-Sympathomimetika (wie z. B. Xylometazolin und Oxymetazolin oder deren physiologisch verträgliche bzw. unbedenkliche Salze), enthalten.

Diese Sympathomimetika führen zwar aufgrund ihrer vasokonstriktorischen Eigenschaften nach lokaler bzw. topischer Anwendung in der Nase zunächst zu einer Nasenschleimhautabschwellung, bewirken jedoch bei wiederholten Anwendung oftmals eine Austrocknung der Nasenschleimhäute, einhergehend mit entzündlichen Irritationen der Nasenschleimhäute (was nicht selten zu einer erhöhten Infektionsgefahr führt, da die Nasenschleimhaut im ausgetrockneten und entzündeten Zustand nicht mehr ihre Schutz- und Filterfunktion im vollen Umfang aufrechterhalten kann und folglich Krankheitserreger ungehindert in die Atemwege gelangen können).

Darüber hinaus wirken die Sympathomimetika weder antibakteriell noch antiviral noch antiallergen. Auch haben die Sympathomimetika zum Teil auch schwerwiegende systemische (z. B. kardiovaskuläre) Nebenwirkungen, insbesondere im Fall von Überdosierung oder bei der Therapierung bestimmter, insbesondere empfindlicher Patienten (z. B. Kleinkindern, Patienten mit kardiovaskulären Vorerkrankungen etc.).

Um den zahlreichen Nebenwirkungen der Sympathomimetika zumindest teilweise entgegenzuwirken und um das zum Teil unzulängliche Wirkprofil der Sympathomimetika bei der Behandlung von Rhinitiden zumindest in gewisser Weise zu verbessern, werden in für die intranasale Applikation bestimmte sympathomimetikahaltigen Zusammensetzungen bisweilen weitere Wirk- bzw. Inhaltsstoffe inkorporiert, beispielsweise pflanzliche Extrakte oder pflanzliche Inhaltsstoffe, befeuchtende oder schleimhautpflegende Zusätze, Antihistaminika, Salze etc. (vgl. beispielsweise DE 195 41 919 A1, DE 195 49 421 A1 und DE 103 56 248 A1).

Darüber hinaus sind aus dem Stand der Technik für die Behandlung von Rhinitiden auch sympathomimetikafreie, für die intranasale Applikation bestimmte Zusammensetzungen bekannt, welche aber oftmals keine ausreichende Wirksamkeit in Bezug auf eine effiziente Behandlung von Rhinitiden aufweisen.

Bei der Behandlung speziell allergischer Rhinitiden, gelegentlich aber auch bei Erkältungsschnupfen kommen zum Teil für die intranasale Applikation bestimmte pharmazeutische Zusammensetzungen zum Einsatz, welche Antihistaminika (Antiallergika) enthalten, wobei viele der topisch eingesetzten Antihistaminika unerwünschte Nebenwirkungen aufweisen, wie insbesondere Müdigkeit, Kopfschmerzen, bitterer Geschmack, Austrocknung der Schleimhäute oder dergleichen.

So betrifft die DE 20 2014 105 553 U1 eine cineolhaltige Zusammensetzung, welche neben Cineol als Wirkkomponente (a) darüber hinaus als Wirkkomponente (b) Pantothenol bzw. dessen physiologisch unbedenkliche Ester und/oder Pantothensäure oder deren physiologisch unbedenkliche Salze enthalten soll.

Zudem betrifft die DE 10 2013 001 151 A1 eine tropf- bzw. sprühbare und bei Raumtemperatur flüssige sowie in der Anwendung gelierende Nasalzubereitung, welche neben Mineralsalzen und Alkoholen sowie Polaxamertensiden noch weitere Zusatzstoffe, wie ätherische Öle, wasserlösliche Vitamine, Enzyme, Proteine und Zinksalze, pH-Wertregulatoren, Konservierungsmittel bzw. Konsistenzmittel, enthält. Als ätherisches Öl kann Eukalyptusöl zum Einsatz kommen.

Was zudem den Einsatz von ätherischen Ölen zu kosmetischen bzw. medizinischen Zwecken im Allgemeinen anbelangt, betrifft die US 6 482 397 B1 eine Zusammensetzung, welche neben einem selbstbräunenden Mittel und einem kosmetisch akzeptablen Träger einen Farbstoff enthalten soll, wobei die Zusammensetzung zur Auftragung auf die Haut geeignet sein soll. In den Zusammensetzungen kann Eukalyptusöl enthalten sein.

Das der vorliegenden Erfindung zugrundeliegende Problem besteht daher in der Bereitstellung einer für die topische, insbesondere nasale, bevorzugt intranasale Applikation, nämlich für die Behandlung von Rhinitiden, geeigneten Zusammensetzung, insbesondere pharmazeutischen Zusammensetzung, welche die zuvor geschilderten Nachteile des Standes der Technik zumindest weitgehend vermeidet oder aber wenigstens abschwächt.

Insbesondere soll eine solche Zusammensetzung bereitgestellt werden, welche gegenüber herkömmlichen, für die Behandlung von Rhinitiden mittels topischer bzw. intranasaler Applikation bestimmten, insbesondere sympathomimetikahaltigen pharmazeutischen Präparaten eine verbesserte Wirkeffizienz und/oder ein verbessertes Nebenwirkungsprofil aufweisen soll.

Weiterhin liegt eine weitere Aufgabe der vorliegenden Erfindung in der Bereitstellung einer für die topische, insbesondere nasale, bevorzugt intranasale Applikation, nämlich für die Behandlung von Rhinitiden, geeigneten Zusammensetzung, welche über eine gute Stabilität, insbesondere eine gute Langzeitstabilität und/oder Lagerstabilität, verfügt.

Zur Lösung des zuvor geschilderten Problems schlägt die vorliegende Erfindung - gemäß einem ersten Aspekt der vorliegenden Erfindung - eine cineolhaltige Zusammensetzung, insbesondere eine pharmazeutische Zusammensetzung, nach Anspruch 1 vor; weitere, insbesondere vorteilhafte Ausgestaltungen der erfindungsgemäßen Zusammensetzung sind Gegenstand der diesbezüglichen Unteransprüche.

Schließlich betrifft die vorliegende Erfindung - gemäß einem weiteren und zweiten Aspekt der vorliegenden Erfindung - ein erfindungsgemäßes Stabilisierungsverfahren (d. h. also ein Verfahren zur Stabilisierung einer cineolhaltigen Zusammensetzung nach der vorliegenden Erfindung), wie es in den entsprechenden Verfahrensansprüchen definiert ist.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100% bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine cineolhaltige Zusammensetzung für die topische Applikation für die Behandlung von Rhinitiden, wobei die Zusammensetzung in Kombination und jeweils in wirksamen Mengen
(a) Cineol, wobei das Cineol als Reinstoff vorliegt und frei von anderen Terpenen ist;
(b) Pantothenol oder dessen physiologisch unbedenkliche Ester und/oder Salze; und
(c) mindestens ein Konservierungsmittel und/oder Desinfektionsmittel (Antiseptikum) in relativen Mengen im Bereich von 0,05 bis 2 Gew.-%, bezogen auf die Zusammensetzung, wobei das Konservierungsmittel und/oder Desinfektionsmittel (Antiseptikum) eine Kombination von Sorbinsäure (Hexa-2,4-diensäure) einerseits und Sorbinsäuresalz (Sorbat) andererseits ("Sorbinsäure/Sorbat-Kombination") umfasst oder hieraus besteht,
enthält,
wobei die Komponente (c) die Kombination von Sorbinsäure einerseits und Sorbat andererseits mit einem Sorbinsäure/Sorbat-Gewichtsverhältnis im Bereich von 9 : 1 bis 1 : 9 umfasst.

Die vorliegende Erfindung ist mit zahlreichen Vorteilen und Besonderheiten verbunden, welche nachfolgend in nicht beschränkender Weise diskutiert sind und als Indiz für Patentfähigkeit zu werten sind.

Im Rahmen der vorliegenden Erfindung wird ein effizient wirkendes Antirhinitismittel (Antirhinitikum) bereitgestellt, welches ohne die zwingende Anwesenheit von alpha-Sympathomimetika und Antihistaminika auskommt, so dass die unerwünschten Nebenwirkungen von alpha-Sympathomimetika und Antihistaminika vermieden werden können.

Die erfindungsgemäße Zusammensetzung besitzt infolge der Anwesenheit der Komponente (a), d. h. des Cineols, insbesondere in Form von 1,8-Cineol, bei deren bestimmungsgemäßer Verwendung nicht nur eine antibakterielle (d. h. bakterizide bzw. bakteriostatische), sondern darüber hinaus - wie die Anmelderin vollkommen überraschend gefunden hat - auch eine antivirale (d. h. viruzide bzw. virustatische) Wirkung.

Weiterhin wirkt die erfindungsgemäße Zusammensetzung entzündungshemmend bzw. antiinflammatorisch und zudem sekretolytisch bzw. schleimlösend. Auf diese Weise kann die erfindungsgemäße Zusammensetzung Entzündungen der Nasenschleimhaut (wie sie bei Rhinitiden stets auftreten) in effizienter Weise entgegenwirken. Zudem bewirkt die erfindungsgemäße Zusammensetzung eine gute Befeuchtung der Nasenschleimhaut.

Auch in Bezug auf allergische Formen der Rhinitis zeigt die erfindungsgemäße Zusammensetzung überraschenderweise eine äußerst positive Wirkung.

Infolge des synergistischen Zusammenwirkens der Komponenten (a) und (b) wird insgesamt eine antirhinitische Wirkung erzielt. Insbesondere wird den Symptomen von Rhinitiden effizient entgegengewirkt, insbesondere auch dem sogenannten Fließschnupfen (Rhinorrhö) (d. h. die erfindungsgemäße Zusammensetzung bzw. Kombination besitzt auch ein antirhinorrhöisches Wirkprofil bzw. Wirkpotential).

Auch wirkt die erfindungsgemäße Zusammensetzung in effizienter Weise der bei Rhinitiden auftretenden Nasenatmungsbehinderung entgegen.

Die erfindungsgemäße Zusammensetzung wirkt aber nicht nur entzündungshemmend bzw. antiinflammatorisch, sondern besitzt insbesondere infolge der Anwesenheit der Komponente (b) auch schleimhautprotektive und wundheilungsfördernde Wirkung, so dass Entzündungen der Nasenschleimhaut, wie sie bei Rhinitiden stets auftreten, effizient entgegengewirkt wirkt. Zudem wirkt die Komponente (b) dahingehend, dass sie die Nasenschleimhaut pflegt und befeuchtet, so dass auf diese Weise auch der Austrocknung der Nasenschleimhaut und einer Borkenbildung entgegengewirkt wird.

Insbesondere aufgrund des antimikrobiellen, insbesondere bakteriziden bzw. bakteriostatischen Wirkpotentials des Cineols wird auch eine gute Stabilität, insbesondere Lagerungs- und Langzeitstabilität, der erfindungsgemäßen Zusammensetzung erreicht. Durch zusätzliche Maßnahmen (z. B. pH-Werteinstellung bzw. pH-Wertkontrolle sowie Verwendung chemischer Puffersysteme, Emulgatoren, Verdickungsmittel etc.) kann die Stabilität der erfindungsgemäßen Zusammensetzung weitergehend verbessert werden. Insbesondere durch die spezielle Auswahl der Komponente (c) wird in vollkommen überraschender Weise eine ausgezeichnete Stabilisierung erreicht.

Denn in Abwesenheit stabilisierender Zusätze bzw. Maßnahmen unterliegen die Wirkstoffkomponenten (a) und (b), insbesondere bei längeren Lagerzeiträumen, einem oxidativen und/oder hydrolytischen Abbau:
So unterliegt in einer flüssigen, insbesondere wässrigen Zusammensetzung der Wirkstoff Cineol einem hydrolytischen und/oder oxidativen Abbau zu den entsprechenden Hydroxy-, Oxo- und/oder Säurederivativen, insbesondere unter Ringöffnung bzw. Öffnung des Epoxidrings.

Weiterhin kann der Wirkstoff Pantothenol in wässriger Lösung hydrolytisch gemäß der nachfolgenden Reaktionsgleichung gespalten werden, wobei sich aus dem Wirkstoff Pantothenol (3) in saurer Lösung - neben dem Ammoniumsalz des 3-Aminopropanols (7) - das Lacton D-Pantolacton (6) als Abbauprodukt bildet, während in alkalischer Lösung - neben 3-Aminopropanol (5) - das Salz der Pantosäure (4) gebildet wird:

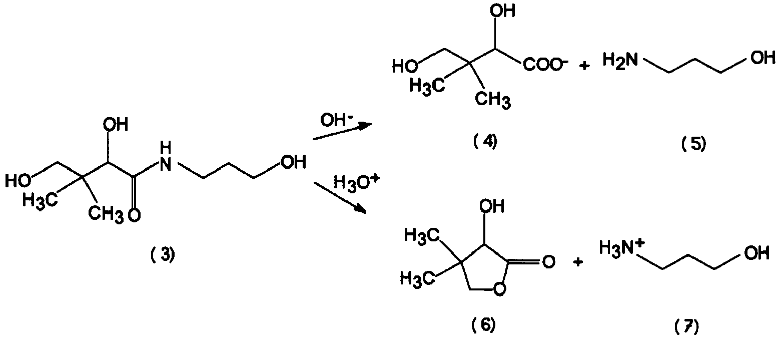

In gemeinsamer, insbesondere wässriger Lösung von Cineol einerseits und Pantothenol andererseits ist eine gleichzeitige Stabilisierung dieser beiden Inhaltsstoffe besonders diffizil, da die einzelnen Wirkstoffe (d. h. Cineol einerseits und Pantothenol andererseits) eine deutlich unterschiedliches lipophiles bzw. hydrophiles Verhalten besitzen und in unterschiedlichen pH-Bereichen ihr Stabilitätsoptimum aufweisen.

In vollkommen überraschenderweise Weise kann aber - trotz dieses stark unterschiedlichen Stabilitätsverhaltens der Komponenten (a) und (b) - eine gleichzeitige Stabilisierung beider Komponenten (a) und (b) und damit der erfindungsgemäßen Zusammensetzung insgesamt in effizienter Weise bewirkt werden, wenn in der Zusammensetzung die zuvor definierte Komponente (c) inkorporiert wird. Mit anderen Worten bewirkt - in vollkommen unerwarteter Weise - ein Konservierungsmittel bzw. Desinfektionsmittel (Antiseptikum) auf Basis einer Kombination, insbesondere einer Mischung, von Sorbinsäure (Hexa-2,4-diensäure) einerseits und Sorbinsäuresalz (Sorbat) andererseits, d. h. also auf Basis einer Sorbinsäure/Sorbat-Kombination, dass beide Komponenten (a) und (b) in gleicher Weise stabilisiert werden können. Im Vergleich zu einer jeweils alleinigen Verwendung von Sorbinsäure oder Sorbat wird dagegen auf Basis einer Sorbinsäure/Sorbat-Kombination überraschenderweise eine signifikante Stabilitätssteigerung der Zusammensetzung erreicht, welche selbst die Stabilisierungswirkung andersartiger Konservierungs- bzw. Desinfektionsmittel (Antiseptika), wie z. B. Benzalkoniumchlorid oder Polyhexanid, übersteigt.

Damit resultiert insgesamt eine für die topische, insbesondere nasale, bevorzugt intranasale Applikation, nämlich für die Behandlung von Rhinitiden, geeignete Zusammensetzung, insbesondere pharmazeutischen Zusammensetzung, welche eine verbesserte Stabilität (z. B. Lagerstabilität bzw. Langzeitstabilität) aufweist.

Durch optionale Inkorporierung weiterer Wirk- und/oder Inhaltsstoffe, insbesondere auf Basis von Emulgatoren (vgl. noch folgende Ausführungen), lassen sich das Wirkprofil und das Stabilitätsverhalten der erfindungsgemäßen Zusammensetzung kontrollieren bzw. gezielt einstellen.

Wie zuvor ausgeführt, enthält die cineolhaltige Zusammensetzung nach der vorliegenden Erfindung als Komponente (a) Cineol, vorzugsweise 1,8-Cineol.

Was den Wirkstoff Cineol, insbesondere 1,8-Cineol, anbelangt, so ist hierzu Folgendes auszuführen: Cineol, insbesondere in Form von 1,8-Cineol, gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist. Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z. B. in Minze, Heilsalbei, Thymian, Basilikum und im Teebaum; darüber hinaus ist 1,8-Cineol beispielsweise in Niaouli-, Juniperus-, Piper-, Cannabis-, Kajeput-, Salbeiöl, Myrtenöl und anderen ätherischen Ölen enthalten. Technisches 1,8-Cineol, welches im Allgemeinen 99,6%-ig bis 99,8%-ig ist, wird im Allgemeinen durch fraktionierte Destillation von Eukalyptusöl gewonnen.

Für weitergehende Einzelheiten zu dem Wirkstoff 1,8-Cineol kann beispielsweise verwiesen werden auf RÖMPP Chemielexikon, Georg Thieme Verlag, Stuttgart/New York, 10. Auflage, Band 1, 1996, Seite 752, Stichwort: "Cineol", sowie auf die dort referierte Literatur.

Wie zuvor bereits ausgeführt, entfaltet das Cineol, insbesondere 1,8-Cineol, in der erfindungsgemäßen Zusammensetzung bei deren bestimmungsgemäßer Verwendung nicht nur eine antibakterielle (d. h. bakterizide bzw. bakteriostatische), sondern darüber hinaus (wie die Anmelderin vollkommen überraschend gefunden hat) auch eine antivirale (d. h. viruzide bzw. virustatische) Wirkung. Weiterhin wirkt das Cineol, insbesondere 1,8-Cineol, im Rahmen der erfindungsgemäßen Zusammensetzung insgesamt entzündungshemmend bzw. antiinflammatorisch und zudem sekretolytisch bzw. schleimlösend; auf diese Weise wirkt 1,8-Cineol - in synergistischer Kombination mit der Komponente (b) (d. h. Pantothenol bzw. Pantothensäure) - Entzündungen der Nasenschleimhaut effizient entgegen und sorgt zudem für eine gute Befeuchtung der Nasenschleimhaut. In synergistischer Zusammenwirkung mit der Komponente (b) wird insgesamt eine antirhinitische Wirkung erzielt und zudem den Symptomen von Rhinitiden effizient entgegengewirkt, insbesondere auch dem sogenannten Fließschnupfen (Rhinorrhö) (d. h. die erfindungsgemäße Zusammensetzung bzw. Kombination besitzt auch ein antirhinorrhöisches Wirkprofil bzw. Wirkpotential). Auch wirkt das Cineol in Zusammenwirken mit der Komponente (b) in effizienter Weise der bei Rhinitiden auftretenden Nasenatmungsbehinderung entgegen. Aufgrund seines antimikrobiellen, insbesondere bakteriziden bzw. bakteriostatischen Wirkpotentials ist das Cineol auch in Bezug auf die Stabilität, insbesondere Lagerungs- und Langzeitstabilität, der erfindungsgemäßen Zusammensetzung förderlich.

Erfindungsgemäß ist es vorgesehen, dass die Komponente (a) das Cineol als Reinstoff, d.h. frei von anderen Terpenen, enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol. Dies bedeutet, dass die Zusammensetzung außer dem Cineol kein weiteres Terpen enthält.

Mit anderen Worten liegt in der cineolhaltigen Zusammensetzung nach der vorliegenden Erfindung das Cineol als Reinstoff vor, d. h. das in der cineolhaltigen Zusammensetzung nach der vorliegenden Erfindung eingesetzte Cineol ist frei von anderen Terpenen bzw. enthält keine anderen Terpene.

In bevorzugter Weise weist das als Komponente (a) verwendete Cineol eine Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, auf.

Gemäß einer besonderen Ausführungsform der vorliegenden Erfindung enthält die erfindungsgemäße Zusammensetzung das Cineol als 1,8-Cineol bzw. liegt das Cineol als 1,8-Cineol vor (d.h. mit anderen Worten enthält gemäß dieser besonderen Ausführungsform die Komponente (a) das Cineol in Form von 1,8-Cineol).

Durch die Verwendung von Cineol als Reinsubstanz, insbesondere in Form von 1,8-Cineol, wird ein definiertes oder steuerbares Wirkprofil gewährleistet. Insbesondere werden infolge der Abwesenheit weiterer Terpene unerwünschte oder unvorhersehbare Wechsel- und Nebenwirkungen vermieden.

Gemäß einer besonderen Ausführungsform der vorliegenden Erfindung kann die Komponente (a) bzw. das Cineol in liposomenumgebener und/oder liposomal verpackter Form vorliegen bzw. formuliert werden. Auf diese Weise wird insbesondere eine verbesserte Löslichkeit bzw. Emulgierbarkeit des Cineols bei gleichzeitig guter Stabilisierung des Cineols in der Zusammensetzung und bei guter Bioverfügbarkeit erreicht.

Die Menge an Komponente (a) bzw. Cineol in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (a) bzw. das Cineol, bezogen auf die Zusammensetzung, in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,5 bis 1,8 Gew.-%, am meisten bevorzugt 0,7 bis 1,5 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt jedoch im Ermessen des Fachmanns.

Im Zusammenhang mit der Applikation bzw. Verabreichung der Komponente (a) (d. h. Cineol, vorzugsweise in Form von 1,8-Cineol) ist es erfindungsgemäß bevorzugt, wenn die Komponente (a) mit einer Einzeldosis im Bereich von 0,01 mg bis 50 mg, insbesondere im Bereich von 0,02 mg bis 25 mg, vorzugsweise im Bereich von 0,05 mg bis 20 mg, bevorzugt im Bereich von 0,05 mg bis 15 mg, verabreicht wird bzw. wenn die Komponente (a) zur Verabreichung mit einer Einzeldosis im Bereich von 0,01 mg bis 50 mg, insbesondere im Bereich von 0,02 mg bis 25 mg, vorzugsweise im Bereich von 0,05 mg bis 20 mg, bevorzugt im Bereich von 0,05 mg bis 15 mg, hergerichtet ist. In Bezug auf die zuvor genannten Einzeldosen ist es erfindungsgemäß zudem üblich, dass 2 bis 10 Einzeldosen pro Tag, insbesondere 3 bis 5 Einzeldosen pro Tag, verabreicht werden, vorzugsweise über den Tag verteilt, bzw. dass die Komponente (a) zur Verabreichung in 2 bis 10 Einzeldosen pro Tag, insbesondere 3 bis 5 Einzeldosen pro Tag, hergerichtet ist, vorzugsweise über den Tag verteilt.

Was die Komponente (b) der erfindungsgemäßen Zusammensetzung anbelangt, so umfasst - wie zuvor bereits dargelegt - diese Komponente (b) Pantothenol, vorzugsweise Dexpanthenol (D-Pantothenol), oder dessen physiologisch unbedenkliche Ester und/oder Salze.

Wie zuvor ausgeführt, enthält also die cineolhaltige Zusammensetzung nach der vorliegenden Erfindung als Komponente (b) Pantothenol, vorzugsweise Dexpanthenol (D-Pantothenol), bzw. dessen physiologisch unbedenkliche Ester und/oder Salze. Der Wirkstoff Pantothenol gehört in chemischer Hinsicht zu den Polyolen und Amiden und ist in physiologischer Hinsicht ein Provitamin (d. h. ein Präkursor eines Vitamins der Vitamin B-Gruppe), welches im Körper zu Pantothensäure (Vitamin B5) umgewandelt wird. Pantothensäure wiederum ist ein Bestandteil des Coenzyms A und spielt damit eine wesentliche Rolle im (Schleim-)-Hautstoffwechsel.

Wie zuvor bereits ausgeführt, entfaltet die Komponente (b) (d. h. Pantothenol, vorzugsweise Dexpanthenol, oder dessen physiologisch unbedenkliche Ester und/oder Salze) in der erfindungsgemäßen Zusammensetzung bei deren bestimmungsgemäßer Verwendung nicht nur eine schleimhautprotektive und wundheilungsfördernde Wirkung, sondern wirkt auch eigenständig entzündungshemmend bzw. antiinflammatorisch. In synergistischer Kombination mit dem Cineol, insbesondere 1,8-Cineol, wird folglich Entzündungen der Nasenschleimhaut, wie sie bei Rhinitiden stets auftreten, effizient entgegengewirkt. Zudem wirkt die Komponente (b) die Nasenschleimhaut pflegend und befeuchtend, so dass auf diese Weise eine gute Befeuchtung der Nasenschleimhaut bewirkt wird bzw. einer Austrocknung der Nasenschleimhaut und einer Borkenbildung entgegengewirkt wird. Auch wird durch die Komponente (b) in Zusammenwirkung mit der Komponente (a) eine verbesserte Nasenatmung erreicht. In synergistischer Zusammenwirkung mit der Komponente (a) wird folglich insgesamt eine antirhinitische Wirkung erzielt und zudem den Symptomen von Rhinitiden effizient entgegengewirkt, insbesondere auch dem Fließschnupfen (Rhinorrhö) (d. h. die erfindungsgemäße Zusammensetzung bzw. Kombination besitzt - wie zuvor angeführt - auch ein antirhinorrhöisches Wirkprofil bzw. Wirkpotential).

Erfindungsgemäß ist es somit vorgesehen, dass die Zusammensetzung als Komponente (b) Pantothenol, vorzugsweise Dexpanthenol (D-Pantothenol), oder dessen physiologisch unbedenkliche Ester enthält
Erfindungsgemäß besonders bevorzugt ist es, wenn die Zusammensetzung als Komponente (b) Pantothenol, bevorzugt in Form von Dexpanthenol (D-Pantothenol), enthält.

Die Menge an Komponente (b) in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (b), bezogen auf die Zusammensetzung, in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Im Zusammenhang mit der Applikation bzw. Verabreichung der Komponente (b) (d. h. Pantothenol bzw. dessen Ester und/oder Salzen) ist es erfindungsgemäß bevorzugt, wenn die Komponente (b), vorzugsweise in Form von Pantothenol, besonders bevorzugt Dexpanthenol, mit einer Einzeldosis im Bereich von 0,01 mg bis 50 mg, insbesondere im Bereich von 0,05 mg bis 25 mg, vorzugsweise im Bereich von 0,1mg bis 20 mg, bevorzugt im Bereich von 0,5 mg bis 10 mg, besonders bevorzugt im Bereich von 1 mg bis 10 mg, verabreicht wird bzw. wenn die Komponente (b), vorzugsweise in Form von Pantothenol, besonders bevorzugt Dexpanthenol, zur Verabreichung mit einer Einzeldosis im Bereich von 0,01 mg bis 50 mg, insbesondere im Bereich von 0,05 mg bis 25 mg, vorzugsweise im Bereich von 0,1 mg bis 20 mg, bevorzugt im Bereich von 0,5 mg bis 10 mg, besonders bevorzugt im Bereich von 1 mg bis 10 mg, hergerichtet ist. In Bezug auf die zuvor genannten Einzeldosen ist es erfindungsgemäß zudem üblich, dass 2 bis 10 Einzeldosen pro Tag, insbesondere 3 bis 5 Einzeldosen pro Tag, verabreicht werden, vorzugsweise über den Tag verteilt, bzw. dass die Komponente (b), vorzugsweise in Form von Pantothenol, besonders bevorzugt Dexpanthenol, zur Verabreichung in 2 bis 10 Einzeldosen pro Tag, insbesondere 3 bis 5 Einzeldosen pro Tag, hergerichtet ist, vorzugsweise über den Tag verteilt.

Für die Wirksamkeit der Komponenten (a) und (b) in der erfindungsgemäßen Zusammensetzung ist auch deren Mengenverhältnis von Bedeutung. Gemäß einer erfindungsgemäß bevorzugten Ausführungsform kann es vorgesehen sein, dass die erfindungsgemäße Zusammensetzung die Komponenten (a) und (b) in einem Mengenverhältnis von Komponente (a) zu Komponente (b) im Bereich von 1 : 1 bis 1 : 500, insbesondere 1 : 1,25 bis 1 : 100, vorzugsweise 1 : 1,5 bis 1 : 75, bevorzugt 1 : 2 bis 1 : 50, besonders bevorzugt 1 : 2,5 bis 1 : 25, ganz besonders bevorzugt 1 : 2,75 bis 1 : 20, noch mehr bevorzugt 1 : 3 bis 1 : 10, enthält. Dennoch kann es einzelfallbedingt oder anwendungsbezogen erforderlich sein, von den vorgenannten Werten abzuweichen, ohne dass der Rahmen der vorliegenden Erfindung verlassen ist (was im Ermessen des Fachmanns liegt).

Was die Komponente (c) der erfindungsgemäßen Zusammensetzung anbelangt, so umfasst - wie zuvor bereits dargelegt - die Komponente (c) mindestens ein Konservierungsmittel und/oder Desinfektionsmittel (Antiseptikum), wobei das Konservierungsmittel und/oder Desinfektionsmittel (Antiseptikum) eine Kombination, insbesondere eine Mischung, von Sorbinsäure (Hexa-2,4-diensäure) einerseits und Sorbinsäuresalz (Sorbat) andererseits ("Sorbinsäure/Sorbat-Kombination") umfasst bzw. enthält oder hieraus besteht.

Ein Konservierungsmittel und/oder Desinfektionsmittel (Antiseptikum) im Sinne der vorliegenden Erfindung ist ein Formulierungshilfsstoff, welcher dazu dient, Mikroorganismen entgegenzuwirken und/oder antimikrobielle bzw. wunddesinfizierende Wirkung zu entfalten. Im Rahmen der vorliegenden Erfindung soll durch die Verwendung der Komponente (c) einem (übermäßigen) Befall der erfindungsgemäßen Zusammensetzung mit Mikroorganismen und somit einer unerwünschten Verkeimung entgegengewirkt werden. Auf diese Weise wird die Stabilität der resultierenden erfindungsgemäßen Zusammensetzung, insbesondere auch im Hinblick auf eine Lagerung über längere Zeiträume, verbessert. Wie die Anmelderin überraschend gefunden hat, unterstützt die Anwesenheit eines Konservierungsmittels und/oder Desinfektionsmittels (Antiseptikums) aber auch das antiinflammatorische und antibakterielle wie antivirale Wirkprofil des Cineols im Rahmen der Applikation der erfindungsgemäßen Zusammensetzung bei der Behandlung von Rhinitiden.

Wie die Anmelderin vollkommen überraschend herausgefunden hat, führt die spezielle Auswahl der zuvor genannten Sorbinsäure/Sorbat-Kombination als Komponente (c) bzw. als Konservierungsmittel und/oder Desinfektionsmittel (Antiseptikum) unerwarteterweise dazu, dass gleichzeitig sowohl die Komponente (a) als auch die Komponente (b) in der erfindungsgemäßen Zusammensetzung wirksam, insbesondere auch über längere Lagerzeiträume, stabilisiert werden und einem Abbau, insbesondere einem oxidativen und/oder hydrolytischen Abbau, dieser beiden Komponenten (a) und (b) in gleicher Weise in effizienter Weise entgegengewirkt werden kann. Dies war für den Fachmann vollkommen überraschend und folglich in keiner Weise zu erwarten. Die Einzelstoffe (d. h. Sorbinsäure einerseits und Sorbat andererseits) erweisen sich dagegen diesbezüglich deutlich weniger wirksam; aber auch andere Konservierungs- oder Desinfektionsmittel (z. B. Benzalkoniumchlorid oder Polyhexanid) haben sich nach Studien der Anmelderin als deutlich weniger wirksam erwiesen.

Bei der erfindungsgemäß zum Einsatz kommenden Sorbinsäure (synonym auch als Hexadiensäure oder Hexa-2,4-Diensäure bezeichnet) handelt es sich um eine zweifach ungesättigte Carbonsäure; sie wird sowohl als freie Säure unter der in der Europäischen Union gültigen Bezeichnung "E 200" als auch in Form ihrer Salze (Sorbate) als Konservierungsmittel verwendet, wobei erfindungsgemäß als Sorbinsäure insbesondere (2E,4E)-Hexa-2,4-Diensäure zum Einsatz kommt. Das Kaliumsorbat ist das Kaliumsalz der Sorbinsäure, welches in der Europäischen Union als Lebensmittelzusatzstoff mit der Nummer "E 202" zugelassen ist. Die Besonderheit der vorliegenden Erfindung besteht in der kombinierten Verwendung von Sorbinsäure einerseits und Sorbinsäuresalzen (Sorbaten) andererseits, einhergehend mit der besonderen Wirkweise im Rahmen der vorliegenden Erfindung.

Die Menge an Komponente (c) in der Zusammensetzung nach der vorliegenden Erfindung kann in weiten Bereichen variieren bzw. eingestellt werden. Erfindungsgemäß ist es vorgesehen, dass die erfindungsgemäße Zusammensetzung die Komponente (c), bezogen auf die Zusammensetzung (und berechnet als Summe aller Bestandteile der Komponente (c)), in relativen Mengen im Bereich von 0,05 bis 2 Gew.-%, ganz besonders bevorzugt 0,05 bis 1 Gew.-%, noch mehr bevorzugt 0,08 bis 0,8 Gew.-%, am meisten bevorzugt 0,1 bis 0,5 Gew.-%, enthält.

Für die Wirksamkeit der Komponenten (c) in der erfindungsgemäßen Zusammensetzung ist auch das Gewichtsverhältnis von Sorbinsäure zu Sorbinsäuresalz (Sorbat) von Bedeutung. Erfindungsgemäß ist es vorgesehen, dass die Komponente (c) der erfindungsgemäßen Zusammensetzung die Kombination von Sorbinsäure einerseits und Sorbat andererseits mit einem Sorbinsäure/Sorbat-Gewichtsverhältnis im Bereich von 9 : 1 bis 1 : 9, insbesondere im Bereich von 8 : 2 bis 2 : 8, vorzugsweise im Bereich von 7 : 3 bis 3 : 7, besonders bevorzugt im Bereich von 6 : 4 bis 4 : 6, ganz besonders bevorzugt im Bereich von 5,5 : 4,5 bis 4,5 : 5,5, noch mehr bevorzugt mit einem Sorbinsäure/Sorbat-Gewichtsverhältnis von etwa 1 : 1, umfasst bzw. enthält.

Was das oder die in der Komponente (c) der erfindungsgemäßen Zusammensetzung eingesetzten Sorbate (Sorbinsäuresalze) anbelangt, so können hier beliebige Metallsalze zum Einsatz kommen, sofern diese Metallsalze physiologisch unbedenklich bzw. physiologisch verträglich und kompatibel in Bezug auf die erfindungsgemäße Zusammensetzung ausgebildet sind. Gemäß einer erfindungsgemäß bevorzugten Ausführungsform kann es vorgesehen sein, dass die Komponente (c) der erfindungsgemäßen Zusammensetzung das Sorbat (Sorbinsäuresalz) in Form eines Alkali- und/oder Erdalkalisalzes, insbesondere in Form eines Alkalisalzes, vorzugsweise in Form eines Natrium- und/oder Kaliumsalzes, besonders bevorzugt in Form von Kaliumsorbat, enthält.

Gemäß einer besonderen Ausführungsform der vorliegenden Erfindung kann es vorgesehen sein, dass die Zusammensetzung nach der vorliegenden Erfindung außerdem als Komponente (d) mindestens einen Emulgator enthält.

Wie die Anmelderin überraschend herausgefunden hat, wird die zusätzliche Inkorporierung einer Emulgatorkomponente (d) zu einer weiterführenden Stabilisierung, insbesondere Langzeitstabilisierung bzw. Stabilisierung bei Lagerung der erfindungsgemäßen Zusammensetzung. Insbesondere sorgt der Emulgator dafür, dass sowohl die Komponente (a) als auch die Komponente (b) stabil in der flüssigen Zusammensetzung, insbesondere in wässriger Umgebung, emulgiert bzw. gelöst bleiben. Dabei werden besonders gute Stabilisierungsergebnisse bei Verwendung der nachfolgend spezifizierten Emulgatoren erhalten, insbesondere bei Verwendung von Ricinusölen (Castorölen) oder Ricinolsäure bzw. deren jeweiligen Derivaten als Emulgatorkomponente (d).

Ein Emulgator im Sinne der vorliegenden Erfindung ist ein Formulierungshilfsstoff, welcher dazu dient, zwei miteinander nicht oder nur geringfügig mischbare Stoffe bzw. Flüssigkeiten zu einem fein verteilten Gemisch (d. h. Emulsion) zu vermengen und zu stabilisieren. Im Rahmen der vorliegenden Erfindung soll insbesondere die Mischbarkeit bzw. Löslichkeit der Komponente (a), d. h. des Cineols, aber gegebenenfalls auch der Komponente (b), mit bzw. in dem wässrigen Exzipienten (Träger) der erfindungsgemäßen Zusammensetzung verbessert und die resultierende Emulsion stabilisiert werden. Auf diese Weise werden aber nicht nur die Mischbarkeit bzw. Emulgierbarkeit der Wirkstoffe in dem wasserbasierten Exzipienten verbessert und die Stabilität der resultierenden erfindungsgemäßen Zusammensetzung erhöht, sondern wird auch die Bioverfügbarkeit der Wirkstoffe, insbesondere des Cineols, verbessert.

Grundsätzlich ist die Verwendung eines Emulgators in Bezug auf die erfindungsgemäße Zusammensetzung zwar optional, stellt aber aus den vorgenannten Gründen eine bevorzugte Ausführungsform dar.

Die Komponente (d) bzw. der Emulgator kann grundsätzlich aus einer Vielzahl unterschiedlichster Verbindungen ausgewählt werden. Gemäß einer bevorzugten Ausführungsform ist es vorgesehen, dass die Komponente (d) bzw. der Emulgator ausgewählt ist aus der Gruppe von (i) gegebenenfalls (poly)ethoxylierten und/oder hydrierten Ricinusölen (= Castorölen) und deren physiologisch unbedenklichen Salzen und/oder Estern; (ii) gegebenenfalls (poly)ethoxylierten und/oder hydrierten Ricinolsäuren und deren physiologisch unbedenklichen Salzen und/oder Estern; sowie deren Mischungen und Kombinationen. Bevorzugterweise ist die Komponente (d) bzw. der Emulgator ausgewählt aus gegebenenfalls (poly)ethoxylierten und/oder hydrierten Ricinusölen (= Castorölen) und deren physiologisch unbedenklichen Salzen und/oder Estern. In besonders bevorzugter Weise ist die Komponente (d) bzw. der Emulgator ausgewählt aus Macrogolglycerolricinoleat.

Ricinusöl ist ein Pflanzenöl, welches aus den Samen des tropischen Wunderbaums (Ricinus communis), eines Wolfsmilchgewächses, gewonnen wird und aus verschiedenen Triglyceriden besteht und in der Pharmazie auch Oleum Ricini s. Castoris, Oleum Ricini virginale, Castoröl, Kastoröl etc. genannt wird. Ricinusöl ist farblos bis leicht gelblich, durchsichtig, dickflüssig, brennbar, schmeckt mild, aber unangenehm und wirkt stark abführend. Der Geruch ist schwach, aber charakteristisch. Das Öl ist mit einer Dichte von 0,922 bis 0,938 g·cm⁻³ eines derjenigen Öle mit der höchsten Dichte. Ricinusöl ist deutlich polar und unterscheidet sich darin von anderen Ölen mit einer guten Löslichkeit in Ethanol, aber nur geringer Mischbarkeit mit aliphatischen Kohlenwasserstoffen. Ricinusöl besteht im Allgemeinen insbesondere zu 80 bis 85 Gew.-% aus dem Triglycerid der Ricinolsäure, auch Triricinolein genannt, daneben insbesondere aus weiteren Glyceriden verschiedener C₁₈-Fettsäuren sowie mehreren flüchtigen Verbindungen. Die Angaben zur Zusammensetzung variieren abhängig von der Herkunft und Literatur. Der Anteil der Fettsäure-Glycerinester liegt bei ca. 77 bis 83 Gew.-% Ricinolsäure, 3 bis 5 Gew.-% Linolsäure, 4 bis 9 Gew.-% Ölsäure, 1 bis 2 Gew.-% Palmitinsäure, 1 bis 3 Gew.-% Stearinsäure sowie geringen Mengen an Vaccensäure, alpha-Linolensäure, Arachinsäure und Eicosensäure. Der Anteil an freien Fettsäuren beträgt etwa 0,75 bis 3,0 Gew.-% und der Wassergehalt 0,25 bis 0,5 Gew.-%, während übrige Verunreinigungen je nach Qualität zwischen 0,01 und 0,2 Gew.-% liegen.

Ricinolsäure (auch synonym als 9Z,12R-12-Hydroxy-9-octadecensäure bezeichnet) ist eine ungesättigte, linear gebaute Omega-9-Fettsäure mit der Summenformel C₁₈H₃₄O₃, welche bei Raumtemperatur als eine ölige, gelbe, in Wasser unlösliche Flüssigkeit vorliegt; Ricinolsäure ist die einzige in größeren Mengen kommerziell verfügbare natürliche Fettsäure, welche eine Hydroxygruppe trägt. Die Gewinnung erfolgt durch Hydrolyse von Rizinusöl, in welchem die Substanz zu 85 bis 92 % in Form von Triglyceriden vorkommt. Ricinolsäure ist verantwortlich für die Wirkungen von Ricinusöl.

Macrogolglycerolricinoleat (vgl. Ph. Eur. [Pharmacopoea Europaea], 8. Ausgabe, Grundwerk 2014, Seiten 3951/3952) enthält hauptsächlich Glycerolricinoleat, ethoxyliert mit 30 bis 50 Molekülen Ethylenoxid (Nominalwert), kleine Mengen Macrogolricinoleat und freier Glykole. Macrogolglycerolricinoleat entsteht durch Reaktion von Ricinusöl mit Ethylenoxid. Macrogolglycerolricinoleat ist kommerziell erhältlich z. B. von der BASF SE (z.B. Kolliphor™ EL bzw. Cremphor™ EL).

Die Menge an Komponente (d) bzw. Emulgator in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (d) und/oder den Emulgator, bezogen auf die Zusammensetzung, in relativen Mengen im Bereich von 0,0001 bis 10 Gew.-%, insbesondere 0,001 bis 8 Gew.-%, vorzugsweise 0,005 bis 6 Gew.-%, bevorzugt 0,01 bis 4 Gew.-%, besonders bevorzugt 0,05 bis 3,5 Gew.-%, ganz besonders bevorzugt 0,1 bis 3 Gew.-%, noch mehr bevorzugt 0,2 bis 2,5 Gew.-%, am meisten bevorzugt 0,5 bis 2 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Des Weiteren kann es vorgesehen sein, dass die Zusammensetzung nach der Erfindung außerdem als Komponente (e) mindestens ein chemisches Puffersystem, insbesondere in Form von Puffersalz(en), enthält.

Zum Begriff des chemischen Puffers bzw. des chemischen Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "*Puffer*", sowie auf die dort referierte Literatur.

Das chemische Puffersystem, insbesondere in Form von Puffersalz(en), dient im Rahmen der vorliegenden Erfindung insbesondere zur Stabilisierung der erfindungsgemäßen Zusammensetzung. Insbesondere führt die Einstellung eines konstanten pH-Werts mittels des Puffersystems überraschenderweise dazu, dass einem unerwünschten Abbau der Wirkstoffe, insbesondere der Komponenten (a) und (b), bei Lagerung auch über einen längeren Zeitraum effizient entgegengewirkt wird. Auf diese Weise wird die Lagerfähigkeit der erfindungsgemäßen Zusammensetzung verbessert.

Die Menge an Komponente (e) bzw. chemischem Puffersystem in der cineolhaltigen Zusammensetzung nach der vorliegenden Erfindung kann in weiten Bereichen variieren bzw. eingestellt werden. Erfindungsgemäß bevorzugt ist es, wenn die erfindungsgemäße Zusammensetzung die Komponente (e) bzw. das chemische Puffersystem, bezogen auf die Zusammensetzung und berechnet als Summe aller Bestandteile des chemischen Puffersystems, in relativen Mengen im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, enthält.

Im Rahmen der vorliegenden Erfindung dient die Komponente (e) bzw. das chemische Puffersystem insbesondere zur Einstellung und/oder Konstanthaltung des pH-Werts der erfindungsgemäßen Zusammensetzung. Wie zuvor ausgeführt, führt dies dazu, dass einem unerwünschten Abbau der Wirkstoffe, insbesondere der Komponenten (a) und (b), bei Lagerung auch über einen längeren Zeitraum effizient entgegengewirkt und auf diese Weise die Lagerfähigkeit der erfindungsgemäßen Zusammensetzung verbessert wird.

In erfindungsgemäß bevorzugter Weise kann es vorgesehen sein, dass die Komponente (e) bzw. das chemische Puffersystem als ein Dihydrogenphosphat/Monohydrogenphosphat-Puffersystem (synonym bisweilen auch als "H₂PO₄⁻HPO₄²⁻-Puffer(system)" bzw. "Phosphatpuffer(system)" bezeichnet), insbesondere als ein Alkalidihydrogenphosphat/Alkalimonohydrogenphosphat-Puffersystem, vorliegt, bevorzugt mit einem Dihydrogenphosphat/Monohydrogenphosphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 200 : 1, noch mehr bevorzugt im Bereich von 7 : 1 bis 100 : 1, ganz besonders bevorzugt im Bereich von 7 : 1 bis 75 : 1, noch mehr bevorzugt im Bereich von 7 : 1 bis 50: 1 und/oder bevorzugt mit einem Dihydrogenphosphat/Monohydrogenphosphat-Gewichtsverhältnis von größer 5 : 1, insbesondere im Bereich von 5 : 1 bis 200 : 1, insbesondere im Bereich von 6: 1 bis 150 : 1, vorzugsweise im Bereich von 7 : 1 bis 100 : 1, besonders bevorzugt im Bereich von 10 : 1 bis 50 : 1. Bei Verwendung dieses speziellen Puffersystems, insbesondere bei gleichzeitiger Einhaltung der vorgenannten Gewichts- und/oder Molverhältnisse, wird einem unerwünschten Abbau der Wirkstoffe, insbesondere der Komponenten (a) und (b), bei Lagerung auch über einen längeren Zeitraum in besonderem Maße entgegengewirkt und wird die Lagerfähigkeit der erfindungsgemäßen Zusammensetzung in besonderem Maße verbessert.

Grundsätzlich kann der pH-Wert der erfindungsgemäßen Zusammensetzung in weiten Bereichen variieren. Erfindungsgemäß bevorzugt ist es jedoch, wenn die erfindungsgemäße Zusammensetzung einen pH-Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, aufweist bzw. wenn der pH-Wert der erfindungsgemäßen Zusammensetzung im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, eingestellt und/oder konstantgehalten wird (vorzugsweise mittels mindestens eines chemischen Puffersystems, insbesondere wie zuvor definiert). Bei Einhaltung der vorgenannten pH-Wertebereiche wird einem unerwünschten Abbau der Wirkstoffe, insbesondere der Komponenten (a) und (b), bei Lagerung auch über einen längeren Zeitraum in besonderem Weise entgegengewirkt und wird auch die Lagerfähigkeit der erfindungsgemäßen Zusammensetzung in besonderem Maße verbessert.

Im Rahmen der vorliegenden Erfindung kann die Bestimmung des pH-Werts mit dem Fachmann an sich bekannten Methoden erfolgen. Insbesondere kann die Bestimmung des pH-Werts gemäß der Methode nach Ph. Eur. [Pharmacopoea Europaea] 7. Ausgabe, Grundwerk 2011, Abschnitt 2.2.3, erfolgen.

Gemäß einer besonderen Ausführungsform der vorliegenden Erfindung kann es vorgesehen sein, dass die erfindungsgemäße Zusammensetzung außerdem als Komponente (f) mindestens ein weiteres Konservierungsmittel und/oder Desinfektionsmittel (Antiseptikum) enthält.

Die Menge an Komponente (f) bzw. an weiterem Konservierungsmittel und/oder Desinfektionsmittel (Antiseptikum) in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (f) bzw. das weitere Konservierungsmittel und/oder Desinfektionsmittel (Antiseptikum), bezogen auf die Zusammensetzung, in relativen Mengen im Bereich von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, noch mehr bevorzugt 0,02 bis 0,1 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Die Komponente (f) bzw. das weitere Konservierungsmittel und/oder Desinfektionsmittel (Antiseptikum) kann grundsätzlich aus einer Vielzahl unterschiedlichster Verbindungen ausgewählt werden. Gemäß einer bevorzugten Ausführungsform kann es vorgesehen sein, dass die Komponente (f) bzw. das weitere Konservierungsmittel und/oder Desinfektionsmittel (Antiseptikum) ausgewählt ist aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) Polyhexanid, (iii) Chlorhexidin, (iv) para-Hydroxybenzoesäureestern und Mischungen von verschiedenen para-Hydroxybenzoesäureestern, vorzugsweise Nipa-Estern, sowie Kombinationen aus zwei oder mehreren der vorgenannten Verbindungen, bevorzugt aus der aus der Gruppe von (i) Alkylbenzyldimethylammoniumchloriden, insbesondere C₃-C₁₈-Alkylbenzyldimethylammoniumchloriden, und Mischungen von verschiedenen Alkylbenzyldimethylammoniumchloriden, vorzugsweise Benzalkoniumchlorid, (ii) Polyhexanid sowie Kombinationen aus zwei oder mehreren der vorgenannten Verbindungen. In besonders bevorzugter Weise ist die Komponente (f) bzw. das weitere Konservierungsmittel und/oder Desinfektionsmittel (Antiseptikum) ausgewählt aus der Gruppe von Benzalkoniumchlorid, Polyhexanid sowie deren Kombinationen. Erfindungsgemäß als Komponente (f) ganz besonders bevorzugt ist Benzalkoniumchlorid (d. h. ein Gemisch von Alkylbenzyldimethylammoniumchloriden, deren Alkylteil aus C₈-C₁₈-Ketten besteht).

Weiterhin kann es vorgesehen sein, dass die erfindungsgemäße Zusammensetzung außerdem als Komponente (g) mindestens ein Verdickungsmittel enthält.

Ein Verdickungsmittel im Sinne der vorliegenden Erfindung ist ein Formulierungshilfsstoff, welcher dazu dient, die Viskosität der erfindungsgemäßen Zusammensetzung zu erhöhen (d. h. die Zusammensetzung zähflüssiger bzw. weniger fließfähig auszubilden). Im Rahmen der vorliegenden Erfindung soll durch die Verwendung der Komponente (g) erreicht werden, dass die erfindungsgemäße Zusammensetzung bei ihrer intranasalen Applikation länger an der Nasenschleimhaut haftet und auf diese Weise die Wirkdauer verlängert wird. Insbesondere wird auf diese Weise die sogenannte Bioadhäsion verbessert. Auch kann auf diese Weise die Wirkstofffreisetzung gezielt kontrolliert werden. Aufgrund der infolge der Anwesenheit des Verdickungsmittels verbesserten mucoadhäsiven Wirkung können eine längere Verweildauer am Wirkort und eine verbesserte Befeuchtung der Nasenschleimhaut erreicht werden.

Die Menge an Komponente (g) bzw. Verdickungsmittel in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (g) bzw. das Verdickungsmittel, bezogen auf die Zusammensetzung, in relativen Mengen im Bereich von 0,0001 bis 10 Gew.-%, insbesondere 0,001 bis 8 Gew.-%, vorzugsweise 0,005 bis 5 Gew.-%, bevorzugt 0,01 bis 3 Gew.-%, besonders bevorzugt 0,05 bis 2 Gew.-%, noch mehr bevorzugt 0,1 bis 1 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Die Komponente (g) bzw. das Verdickungsmittel kann grundsätzlich aus einer Vielzahl unterschiedlichster Verbindungen ausgewählt werden. Gemäß einer bevorzugten Ausführungsform kann es vorgesehen sein, dass die Komponente (g) bzw. das Verdickungsmittel ausgewählt ist aus der Gruppe von (i) vorzugsweise sauren Glykosaminoglykanen oder deren physiologisch unbedenkliche Salzen oder Derivaten, insbesondere Hyaluronsäuren oder deren physiologisch unbedenklichen Salzen (Hyaluronaten), (ii) Polysacchariden und Hydrokolloiden, insbesondere Carrageenen (Carrageenanen) (iii) Cellulosen und Cellulosederivaten, insbesondere Celluloseestern und Celluloseethern, (iv) Alginsäuren und deren Salzen (Alginaten), (v) Poly(meth)acrylsäuren und Poly(meth)acrylaten, (vi) Polyalkylenglykolen, insbesondere Polyethylenglykolen, (vii) Xanthanen sowie Kombinationen aus zwei oder mehreren der vorgenannten Verbindungen.

In besonders bevorzugter Weise ist die Komponente (g) bzw. das Verdickungsmittel ausgewählt aus der aus der Gruppe von Hyaluronsäuren oder deren physiologisch unbedenklichen Salze (Hyaluronaten), Carrageenen (Carrageenanen) sowie deren Kombinationen.

Für den Fall, dass als Komponente (g) bzw. als Verdickungsmittel eine Cellulose oder Cellulosederivat verwendet wird, kommt insbesondere Hydroxypropylmethylcellulose (synonym auch als "HPMC", "Hypromellose" etc. bezeichnet) oder deren Derivate (insbesondere Ether und/oder Ester) zum Einsatz. Hydroxypropylmethylcellulose (HPMC) ist ein Stoffgemisch aus verschiedenen, teilweise alkylsubstituierten Cellulosen. Sie ist in verschiedenen Polymerisationsgraden und unterschiedlichen Substitutionsgraden kommerziell verfügbar.

Weiterhin kann es vorgesehen sein, dass die erfindungsgemäße Zusammensetzung außerdem als Komponente (h) mindestens ein Ectoin oder Ectoinderivat, insbesondere ein Hydroxyectoin, enthält, insbesondere in relativen Mengen im Bereich von 0,0001 bis 10 Gew.-%, insbesondere 0,001 bis 5 Gew.-%, vorzugsweise 0,01 bis 2 Gew.-%, bezogen auf die Zusammensetzung.

Gleichermaßen kann es vorgesehen sein, dass die erfindungsgemäße Zusammensetzung außerdem als Komponente (j) mindestens ein vorzugsweise imidazolinbasiertes alpha-Sympathomimetikum oder dessen physiologisch unbedenkliche Salze enthält; dies ist insbesondere der Fall, wenn zusätzlich eine dekongestive bzw. abschwellende Wirkung der erfindungsgemäßen Zusammensetzung im besonderen Maße gewünscht ist (auch wenn die Anwesenheit eines alpha-Sympathomimetikums für den Therapieerfolg der erfindungsgemäßen Zusammensetzung grundsätzlich nicht erforderlich ist). Bei dieser Ausführungsform kann das vorzugsweise imidazolinbasierte alpha-Sympathomimetikum insbesondere ausgewählt sein aus Xylometazolin oder Oxymetazolin, insbesondere in Form von deren physiologisch unbedenklichen Salzen, besonders bevorzugt in Form von deren Hydrochloridsalzen, und besonders bevorzugt Xylometazolinhydrochlorid sein. Bei dieser Ausführungsform kann die erfindungsgemäße Zusammensetzung das vorzugsweise imidazolinbasierte alpha-Sympathomimetikum, bezogen auf die Zusammensetzung, in relativen Mengen im Bereich von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthalten.

Darüber hinaus kann es vorgesehen sein, dass die erfindungsgemäße Zusammensetzung außerdem Natriumchlorid enthält, insbesondere in relativen Mengen im Bereich von 0,001 bis 5 Gew.-%, insbesondere 0,01 bis 2 Gew.-%, vorzugsweise 0,1 bis 1 Gew.-%, bezogen auf die Zusammensetzung. Dies kann insbesondere der Fall sein, wenn eine zusätzliche Befeuchtung der Nasenschleimhaut beabsichtigt ist.

Gemäß einer besonderen Ausführungsform betrifft die vorliegende Erfindung eine cineolhaltige Zusammensetzung, insbesondere eine pharmazeutische Zusammensetzung, welche für die topische, insbesondere nasale, bevorzugt intranasale Applikation, in der vorliegenden Erfindung für die Behandlung von Rhinitiden, geeignet ist, insbesondere eine wie zuvor beschriebene Zusammensetzung nach der vorliegenden Erfindung,
wobei die Zusammensetzung in Kombination und jeweils in wirksamen, insbesondere pharmazeutisch wirksamen Mengen enthält:
(a) Cineol, vorzugsweise 1,8-Cineol, insbesondere in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,5 bis 1,8 Gew.-%, am meisten bevorzugt 0,7 bis 1,5 Gew.-%;
(b) Pantothenol, vorzugsweise Dexpanthenol (D-Pantothenol), oder dessen physiologisch unbedenkliche Ester und/oder Salze, insbesondere in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%;
(c) mindestens ein Konservierungsmittel und/oder Desinfektionsmittel (Antiseptikum), wobei das Konservierungsmittel und/oder Desinfektionsmittel (Antiseptikum) eine Kombination, insbesondere eine Mischung, von Sorbinsäure (Hexa-2,4-diensäure) einerseits und Sorbinsäuresalz (Sorbat) andererseits ("Sorbinsäure/Sorbat-Kombination") umfasst oder hieraus besteht, in relativen Mengen im Bereich von 0,05 bis 2 Gew.-%, ganz besonders bevorzugt 0,05 bis 1 Gew.-%, noch mehr bevorzugt 0,08 bis 0,8 Gew.-%, am meisten bevorzugt 0,1 bis 0,5 Gew.-%;
(d) gegebenenfalls mindestens einen Emulgator, insbesondere in relativen Mengen im Bereich von 0,0001 bis 10 Gew.-%, insbesondere 0,001 bis 8 Gew.-%, vorzugsweise 0,005 bis 6 Gew.-%, bevorzugt 0,01 bis 4 Gew.-%, besonders bevorzugt 0,05 bis 3,5 Gew.-%, ganz besonders bevorzugt 0,1 bis 3 Gew.-%, noch mehr bevorzugt 0,2 bis 2,5 Gew.-%, am meisten bevorzugt 0,5 bis 2 Gew.-%;
(e) gegebenenfalls mindestens ein chemisches Puffersystem, insbesondere in Form von Puffersalz(en), insbesondere in relativen Mengen, berechnet als Summe aller Bestandteile des chemischen Puffersystems, im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%;
(f) gegebenenfalls mindestens ein weiteres Konservierungsmittel und/oder Desinfektionsmittel (Antiseptikum), insbesondere in relativen Mengen im Bereich von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, noch mehr bevorzugt 0,02 bis 0,1 Gew.-%;
(g) gegebenenfalls mindestens ein Verdickungsmittel, insbesondere in relativen Mengen im Bereich von 0,0001 bis 10 Gew.-%, insbesondere 0,001 bis 8 Gew.-%, vorzugsweise 0,005 bis 5 Gew.-%, bevorzugt 0,01 bis 3 Gew.-%, besonders bevorzugt 0,05 bis 2 Gew.-%, noch mehr bevorzugt 0,1 bis 1 Gew.-%;
(h) gegebenenfalls mindestens ein Ectoin oder Ectoinderivat, insbesondere ein Hydroxyectoin, insbesondere in relativen Mengen im Bereich von 0,0001 bis 10Gew.-%, insbesondere 0,001 bis 5 Gew.-%, vorzugsweise 0,01 bis 2 Gew.-%;
(i) gegebenenfalls mindestens ein vorzugsweise imidazolinbasiertes alpha-Sympathomimetikum oder dessen physiologisch unbedenkliche Salze, insbesondere in relativen Mengen im Bereich von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%
(j) Natriumchlorid, insbesondere in relativen Mengen im Bereich von 0,001 bis 5 Gew.-%, insbesondere 0,01 bis 2 Gew.-%, vorzugsweise 0,1 bis 1 Gew.-%;
wobei alle vorgenannten relativen Mengenangaben auf die Zusammensetzung bezogen sind.

Gemäß einer weiteren besonderen Ausführungsform betrifft die vorliegende Erfindung eine cineolhaltige Zusammensetzung, insbesondere eine pharmazeutische Zusammensetzung, welche für die topische, insbesondere nasale, bevorzugt intranasale Applikation, in der vorliegenden Erfindung für die Behandlung von Rhinitiden, geeignet ist, insbesondere eine wie zuvor beschriebene Zusammensetzung nach der vorliegenden Erfindung,
wobei die Zusammensetzung in Kombination und jeweils in wirksamen, insbesondere pharmazeutisch wirksamen Mengen enthält:
(a) Cineol, vorzugsweise 1,8-Cineol, insbesondere in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,5 bis 1,8 Gew.-%, am meisten bevorzugt 0,7 bis 1,5 Gew.-%;
(b) Pantothenol, vorzugsweise Dexpanthenol (D-Pantothenol), oder dessen physiologisch unbedenkliche Ester und/oder Salze, insbesondere in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%;
(c) mindestens ein Konservierungsmittel und/oder Desinfektionsmittel (Antiseptikum), wobei das Konservierungsmittel und/oder Desinfektionsmittel (Antiseptikum) eine Kombination, insbesondere eine Mischung, von Sorbinsäure (Hexa-2,4-diensäure) einerseits und Sorbinsäuresalz (Sorbat) andererseits ("Sorbinsäure/Sorbat-Kombination") umfasst oder hieraus besteht, in relativen Mengen im Bereich von 0,05 bis 2 Gew.-%, ganz besonders bevorzugt 0,05 bis 1 Gew.-%, noch mehr bevorzugt 0,08 bis 0,8 Gew.-%, am meisten bevorzugt 0,1 bis 0,5 Gew.-%;
(d) gegebenenfalls mindestens einen Emulgator, insbesondere in relativen Mengen im Bereich von 0,0001 bis 10 Gew.-%, insbesondere 0,001 bis 8 Gew.-%, vorzugsweise 0,005 bis 6 Gew.-%, bevorzugt 0,01 bis 4 Gew.-%, besonders bevorzugt 0,05 bis 3,5 Gew.-%, ganz besonders bevorzugt 0,1 bis 3 Gew.-%, noch mehr bevorzugt 0,2 bis 2,5 Gew.-%, am meisten bevorzugt 0,5 bis 2 Gew.-%;
(e) gegebenenfalls mindestens ein chemisches Puffersystem, insbesondere in Form von Puffersalz(en), insbesondere in relativen Mengen, berechnet als Summe aller Bestandteile des chemischen Puffersystems, im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%;
wobei alle vorgenannten relativen Mengenangaben auf die Zusammensetzung bezogen sind.
Gemäß einer wiederum weiteren besonderen Ausführungsform betrifft die vorliegende Erfindung eine cineolhaltige Zusammensetzung, insbesondere eine pharmazeutische Zusammensetzung, welche für die topische, insbesondere nasale, bevorzugt intranasale Applikation, in der vorliegenden Erfindung für die Behandlung von Rhinitiden, geeignet ist, insbesondere eine wie zuvor beschriebene Zusammensetzung nach der vorliegenden Erfindung,
wobei die Zusammensetzung in Kombination und jeweils in wirksamen, insbesondere pharmazeutisch wirksamen Mengen enthält:
(a) 1,8-Cineol in relativen Mengen im Bereich von 0,2 bis 2 Gew.-%;
(b) Dexpanthenol (D-Pantothenol) in relativen Mengen im Bereich von 2 bis 6 Gew.-%;
(c) mindestens ein Konservierungsmittel und/oder Desinfektionsmittel (Antiseptikum) in relativen Mengen im Bereich von 0,05 bis 1 Gew.-%, wobei das Konservierungsmittel und/oder Desinfektionsmittel (Antiseptikum) eine Kombination, insbesondere eine Mischung, von Sorbinsäure (Hexa-2,4-diensäure) einerseits und Sorbinsäuresalz (Sorbat) andererseits ("Sorbinsäure/Sorbat-Kombination") mit einem Sorbinsäure/Sorbat-Gewichtsverhältnis im Bereich von 6 : 4 bis 4 : 6 umfasst oder hieraus besteht;
(d) mindestens einen Emulgator in relativen Mengen im Bereich von 0,1 bis 3 Gew.-%, wobei der Emulgator ausgewählt ist aus der Gruppe von (i) gegebenenfalls (poly)ethoxylierten und/oder hydrierten Ricinusölen (Castorölen) und deren physiologisch unbedenklichen Salzen und/oder Estern; (ii) gegebenenfalls (poly)ethoxylierten und/oder hydrierten Ricinolsäuren und deren physiologisch unbedenklichen Salzen und/oder Estern; sowie deren Mischungen und Kombinationen, bevorzugt gegebenenfalls (poly)-ethoxylierten und/oder hydrierten Ricinusölen (Castorölen) und deren physiologisch unbedenklichen Salzen und/oder Estern, besonders bevorzugt Macrogolglycerolricinoleat;
(e) mindestens ein chemisches Puffersystem, insbesondere in Form von Puffersalz(en) in relativen Mengen, berechnet als Summe aller Bestandteile des chemischen Puffersystems, im Bereich von 0,001 bis 4 Gew.-%, wobei das chemische Puffersystem als ein Dihydrogenphosphat/Monohydrogenphosphat-Puffersystem vorliegt;
wobei alle vorgenannten relativen Mengenangaben auf die Zusammensetzung bezogen sind.

Die cineolhaltige Zusammensetzung nach der vorliegenden Erfindung kann grundsätzlich in unterschiedlichsten Erscheinungsformen vorliegen und/oder in unterschiedlichsten Formulierungsformen ausgestaltet sein.

Im Allgemeinen kann die Zusammensetzung nach der vorliegenden Erfindung als wässrige Zusammensetzung vorliegen. Insbesondere kann die Zusammensetzung nach der vorliegenden Erfindung wässrig basiert sein und/oder wässrig formuliert vorliegen, insbesondere in Form einer wässrigen Lösung oder wässrigen Solubilisierung. Auf diese Weise werden eine gute physiologische Kompatibilität und eine gute Applizierbarkeit sichergestellt.

Gemäß einer bevorzugten Ausführungsform kann die erfindungsgemäße cineolhaltige Zusammensetzung als wässriges System, insbesondere als wässriges Einphasensystem, vorzugsweise als wässrige Lösung oder wässrige Solubilisierung, vorliegen. Im Allgemeinen weist die Zusammensetzung nach der vorliegenden Erfindung einen Exzipienten oder Träger auf Wasserbasis auf. Bevorzugterweise liegt die Zusammensetzung nach der Erfindung als klare, farblose wässrige Lösung vor. Dies gewährleistet einerseits eine gute physiologische Verträglichkeit sowie eine gute Anwendbarkeit und andererseits eine gute Stabilität, insbesondere Lagerstabilität, und zwar auch über lange Zeiträume.

Im Rahmen der vorliegenden Erfindung kann die Bestimmung des Aussehens der erfindungsgemäßen Zusammensetzung mit dem Fachmann an sich bekannten Methoden erfolgen. Insbesondere kann die Bestimmung des Aussehens der erfindungsgemäßen Zusammensetzung gemäß der Methode nach Ph. Eur. [Pharmacopoea Europaea] 7. Ausgabe, Grundwerk 2011, Abschnitte 2.2.1 und 2.2.2, erfolgen.

Auch die Osmolalität der cineolhaltigen Zusammensetzung nach der vorliegenden Erfindung kann in weiten Grenzen variieren. Zur Gewährleistung einer guten physiologischen Kompatibilität bei gleichzeitig guter Applizierbarkeit hat es sich bewährt, wenn die erfindungsgemäße Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist. Dennoch kann es einzelfallbedingt oder anwendungsbezogen erforderlich sein, von den vorgenannten Werten abzuweichen, ohne dass der Rahmen der vorliegenden Erfindung verlassen ist; dies liegt im freien Ermessen des auf diesem Gebiet tätigen Fachmanns.

Im Rahmen der vorliegenden Erfindung kann die Bestimmung der Osmolalität der erfindungsgemäßen Zusammensetzung mit dem Fachmann an sich bekannten Methoden erfolgen. Insbesondere kann die Bestimmung der Osmolalität der erfindungsgemäßen Zusammensetzung gemäß der Methode nach Ph. Eur. [Pharmacopoea Europaea] 7. Ausgabe, Grundwerk 2011, Abschnitt 2.2.35, Kap. 5.3.9, erfolgen.

Ebenfalls kann die Dichte der cineolhaltigen Zusammensetzung nach der vorliegenden Erfindung in weiten Bereichen variieren. Insbesondere kann es vorgesehen sein, dass die erfindungsgemäße cineolhaltige Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist. Dennoch kann es einzelfallbedingt oder anwendungsbezogen erforderlich sein, von den vorgenannten Werten abzuweichen, ohne dass der Rahmen der vorliegenden Erfindung verlassen ist.

Im Rahmen der vorliegenden Erfindung kann die Bestimmung der relativen Dichte der erfindungsgemäßen Zusammensetzung mit dem Fachmann an sich bekannten Methoden erfolgen. Insbesondere kann die Bestimmung der relativen Dichte der erfindungsgemäßen Zusammensetzung gemäß der Methode nach Ph. Eur. [Pharmacopoea Europaea] 7. Ausgabe, Grundwerk 2011, Abschnitt 2.2.5, erfolgen.

Anwendungsabhängig kann auch die Viskosität der cineolhaltigen Zusammensetzung nach der vorliegenden Erfindung in weiten Bereichen eingestellt bzw. ausgewählt sein.

Gemäß einer besonderen Ausführungsform, wonach die erfindungsgemäße Zusammensetzung in Abwesenheit eines Verdickungsmittels vorliegt (d. h. wenn die erfindungsgemäße Zusammensetzung ohne ein Verdickungsmittel formuliert ist bzw. kein Verdickungsmittel enthält), kann die Zusammensetzung nach der vorliegenden Erfindung bei einer Temperatur von 20 °C eine dynamische Viskosität im Bereich von 1,001 bis 2 mPas, insbesondere 1,01 bis 1,9 mPas, vorzugsweise 1,05 bis 1,8 mPas, besonders bevorzugt 1,1 bis 1,5 mPas, aufweisen (wobei die Bestimmung der dynamischen Viskosität insbesondere gemäß der Methode nach Ph. Eur. [Pharmacopoea Europaea] 7. Ausgabe, Grundwerk 2011, Abschnitt 2.2.9 Kapillarviskosimeter [bevorzugt Ubbelohde-Viskosimeter], erfolgen kann). Eine solche Ausführungsform ist insbesondere dann bevorzugt, wenn eine gute Applizierbarkeit der Zusammensetzung mittels Sprühauftrag im Vordergrund steht.

Gemäß einer weiteren, hierzu alternativen besonderen Ausführungsform, wonach die erfindungsgemäße Zusammensetzung in Anwesenheit eines Verdickungsmittels vorliegt (d. h. wenn die erfindungsgemäße Zusammensetzung mit einem Verdickungsmittel formuliert ist bzw. ein Verdickungsmittel enthält), kann die Zusammensetzung nach der vorliegenden Erfindung bei einer Temperatur von 20 °C eine dynamische Viskosität von mindestens 1,5 mPas, insbesondere im Bereich von 1,5 bis 10⁵ mPas, vorzugsweise 1,6 bis 10⁴ mPas, besonders bevorzugt 1,7 bis 10³ mPas, noch mehr bevorzugt 2 bis 100 mPas, aufweisen (wobei die Bestimmung der dynamischen Viskosität insbesondere gemäß der Methode nach Ph. Eur. [Pharmacopoea Europaea] 7. Ausgabe, Grundwerk 2011, Abschnitt 2.2.9 Kapillarviskosimeter [bevorzugt Ubbelohde-Viskosimeter], erfolgen kann). Eine solche Ausführungsform ist insbesondere dann bevorzugt, wenn eine verlängerte Wirkungsdauer und/oder eine verbesserte Bioadhäsion der Zusammensetzung im Vordergrund steht.

Ebenfalls kann der Brechungsindex der cineolhaltigen Zusammensetzung nach der vorliegenden Erfindung in weiten Bereichen variieren. Insbesondere kann es vorgesehen sein, dass die erfindungsgemäße cineolhaltige Zusammensetzung bei einer Temperatur von 20 °C und bei einem Druck von 1.013,25 mbar (Atmosphärendruck) einen Brechungsindex im Bereich von 1,10 bis 1,50, insbesondere im Bereich von 1,20 bis 1,40, vorzugsweise im Bereich von 1,25 bis 1,39, aufweist. Dennoch kann es einzelfallbedingt oder anwendungsbezogen erforderlich sein, von den vorgenannten Werten abzuweichen, ohne dass der Rahmen der vorliegenden Erfindung verlassen ist.

Im Rahmen der vorliegenden Erfindung kann die Brechungsindexbestimmung der erfindungsgemäßen Zusammensetzung mit dem Fachmann an sich bekannten Methoden erfolgen. Insbesondere kann die Brechungsindexbestimmung der erfindungsgemäßen Zusammensetzung gemäß der Methode nach Ph. Eur. [Pharmacopoea Europaea] 7. Ausgabe, Grundwerk 2011, Abschnitt 2.2.6, erfolgen.

Wie zuvor bereits ausgeführt, verfügt die cineolhaltige Zusammensetzung nach der vorliegenden Erfindung über eine gute Stabilität, insbesondere Lager- und Langzeitstabilität. Gemäß einer besonderen Ausführungsform ist die Zusammensetzung bei Temperaturen im Bereich von 10 °C bis 40 °C, insbesondere im Bereich von 20 °C bis 30 °C, und bei einem Druck von 1.013,25 mbar (Atmosphärendruck) und bei einer relativen Luftfeuchtigkeit im Bereich von 50 % bis 90 %, insbesondere im Bereich von 50 % bis 75 %, mindestens 6 Monate, insbesondere mindestens 12 Monate, vorzugsweise mindestens 24 Monate, bevorzugt mindestens 36 Monate, stabil, insbesondere lagerstabil, ausgebildet.

Des Weiteren kann es erfindungsgemäß vorgesehen sein, dass die erfindungsgemäße cineolhaltige Zusammensetzung mindestens einen weiteren Inhaltsstoff, insbesondere Hilfsstoff und/oder Additiv, aufweist. Der weitere Inhaltsstoff, insbesondere Hilfsstoff und/oder Additiv, kann insbesondere ausgewählt sein aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen (d. h. Kombinationen von zwei oder mehreren der vorgenannten Inhaltsstoffen).

Wie eingangs dargelegt, verfügt die cineolhaltige Zusammensetzung nach der vorliegenden Erfindung über ein breites Anwendungsprofil. So betrifft die vorliegende Erfindung eine wie zuvor geschilderte cineolhaltige Zusammensetzung zur Verwendung bei der prophylaktischen und/oder kurativen topischen Behandlung von Rhinitiden, insbesondere von *Rhinitis acuta* oder von *Rhinitis allergica.*

Erfindungsgemäß kann es vorgesehen sein, dass eine Applikationsvorrichtung-eine wie zuvor beschriebene cineolhaltige Zusammensetzung nach der vorliegenden Erfindung enthält, wobei die Applikationsvorrichtung insbesondere für die topische, insbesondere nasale, vorzugsweise intranasale Applikation geeignet ist und bevorzugt in Form eines Behältnisses mit Tropf- oder Sprüheinrichtung vorliegt. Erfindungsgemäß bevorzugt ist es, wenn die Applikationsvorrichtung eine Sprüheinrichtung zur vorzugsweise gleichförmigen Ausbringung der Zusammensetzung nach der vorliegenden Erfindung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Dabei ist es insbesondere vorgesehen, dass die Applikationsvorrichtung ein Behältnis bzw. einen Vorratsbehälter mit einem Volumen im Bereich von 5 ml bis 100 ml, insbesondere 10 ml bis 50 ml, aufweist. Dieses Behältnis bzw. dieser Vorratsbehälter dient dann zur Aufnahme der erfindungsgemäßen Zusammensetzung.

Die erfindungsgemäße cineolhaltige Zusammensetzung eignet sich zur die Verwendung bei der prophylaktischen und/oder kurativen topischen Behandlung von Rhinitiden aller Art, insbesondere von *Rhinitis acuta* oder *Rhinitis allergica,* bzw. zur Herstellung eines Arzneimittels zur prophylaktischen und/oder kurativen topischen Behandlung von Rhinitiden aller Art, insbesondere von *Rhinitis acuta* oder *Rhinitis allergica.*

Der Begriff des Arzneimittels (synonym auch Pharmazeutikum), wie er im Rahmen der vorliegenden Erfindung verwendet wird, ist sehr umfänglich zu verstehen und umfasst nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel sowie Kosmetika und Gebrauchsgegenstände. Mit anderen Worten kann also die erfindungsgemäße cineolhaltige Zusammensetzung in Form eines Arzneimittels (Pharmazeutikums), Medizinprodukts, Homöopathikums, Nahrungsergänzungsmittels, Kosmetikums oder Gebrauchsgegenstands vorliegen.

Insbesondere kann die erfindungsgemäße Zusammensetzung zur Behandlung von Rhinitiden aller Art, insbesondere *Rhinitis acuta*, *Rhinitis allergica*, *Rhinitis atrophicans*, *Rhinitis hyperplastica* oder *hypertrophicans*, *Rhinitis mutilans*, *Rhinitis nervo*sa oder *vasomotorica*, umweltbedingter Rhinitis oder *Rhinitis pseudomem*branacea, vorzugsweise *Rhinitis acuta* oder *Rhinitis allergica,* eingesetzt werden.

Wie zuvor beschrieben, kann im Rahmen der Verwendung die erfindungsgemäße Zusammensetzung, wie zuvor beschrieben, topisch, insbesondere nasal, vorzugsweise intranasal, appliziert werden. Dabei kann insbesondere die Applikationseinrichtung, wie sie zuvor beschrieben worden ist, zur Anwendung kommen.

Weiterer Gegenstand der vorliegenden Erfindung - gemäß einem **zweiten** Aspekt der vorliegenden Erfindung - ist schließlich ein Verfahren zur Stabilisierung einer cineolhaltigen Zusammensetzung für die topische Applikation für die Behandlung von Rhinitiden,
wobei die Zusammensetzung in Kombination und jeweils in pharmazeutisch wirksamen Mengen
(a) Cineol, wobei das Cineol als Reinstoff vorliegt und frei von anderen Terpenen ist;
(b) Pantothenol oder dessen physiologisch unbedenkliche Ester und/oder Salze; enthält,
**dadurch gekennzeichnet,**
dass der cineolhaltigen Zusammensetzung als Komponente (c) in relativen Mengen im Bereich von 0,05 bis 2 Gew.-%, bezogen auf die Zusammensetzung mindestens ein Konservierungsmittel und/oder Desinfektionsmittel (Antiseptikum) zugesetzt wird, wobei das Konservierungsmittel und/oder Desinfektionsmittel (Antiseptikum) eine Kombination von Sorbinsäure (Hexa-2,4-diensäure) einerseits und Sorbinsäuresalz (Sorbat) andererseits ("Sorbinsäure/Sorbat-Kombination") umfasst oder hieraus besteht und wobei die Komponente (c) die Kombination von Sorbinsäure einerseits und Sorbat andererseits mit einem Sorbinsäure/Sorbat-Gewichtsverhältnis im Bereich von 9 : 1 bis 1 : 9 umfasst.

Wie zuvor im Rahmen der übrigen Erfindungsaspekte ausgeführt, wird im Rahmen des erfindungsgemäßen Verfahrens (Stabilisierungsverfahrens) der Zusammensetzung die Komponente (c), bezogen auf die Zusammensetzung, in relativen Mengen im Bereich von 0,05 bis 2 Gew.-%, ganz besonders bevorzugt 0,05 bis 1 Gew.-%, noch mehr bevorzugt 0,08 bis 0,8 Gew.-%, am meisten bevorzugt 0,1 bis 0,5 Gew.-%, zugesetzt.

Wie gleichermaßen zuvor im Rahmen der übrigen Erfindungsaspekte ausgeführt, umfasst im Rahmen des erfindungsgemäßen Verfahrens die Komponente (c) die Kombination von Sorbinsäure einerseits und Sorbat andererseits insbesondere mit einem Sorbinsäure/Sorbat-Gewichtsverhältnis im Bereich von 9 : 1 bis 1 : 9, insbesondere im Bereich von 8 : 2 bis 2 : 8, vorzugsweise im Bereich von 7 : 3 bis 3 : 7, besonders bevorzugt im Bereich von 6 : 4 bis 4 : 6, ganz besonders bevorzugt im Bereich von 5,5:4,5 bis 4,5: 5,5, noch mehr bevorzugt mit einem Sorbinsäure/Sorbat-Gewichtsverhältnis von etwa 1 : 1.

Wie ebenfalls zuvor im Rahmen der übrigen Erfindungsaspekte ausgeführt, kann im Rahmen des erfindungsgemäßen Verfahrens die Komponente (c) das Sorbat (Sorbinsäuresalz) in Form eines Alkali- und/oder Erdalkalisalzes, insbesondere in Form eines Alkalisalzes, vorzugsweise in Form eines Natrium- und/oder Kaliumsalzes, besonders bevorzugt in Form von Kaliumsorbat, enthalten.

Wie schließlich ebenfalls zuvor im Rahmen der übrigen Erfindungsaspekte ausgeführt, kann im Rahmen des erfindungsgemäßen Verfahrens der Zusammensetzung außerdem als Komponente (d) mindestens ein Emulgator zugesetzt werden (insbesondere wie vorstehend im Rahmen der übrigen Erfindungsaspekte definiert und/oder insbesondere in Mengen wie im Rahmen der übrigen Erfindungsaspekte definiert).

Für weitergehende Einzelheiten zu dem erfindungsgemäßen Verfahren (Stabilisierungsverfahren) kann auf die vorangehenden Ausführungen zu den übrigen Erfindungsaspekten verwiesen werden, welche in Bezug auf das erfindungsgemäße Verfahren entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellungsbeispiele sowie Stabilitäts- und Wirkverhalten Allgemeine Herstellvorschrift

Zur Herstellung von jeweils 1.000 g einer klaren wässrigen Lösung der erfindungsgemäßen Zusammensetzung wird in dem Fachmann an sich bekannter Weise vorgegangen: Zunächst wird bei Raumtemperatur und Umgebungsdruck in einem entsprechenden Glasgefäß mit Rühreinrichtung eine definierte Menge an gereinigtem Wasser vorgelegt und nachfolgend mit einer Lösung des gewählten Puffersystems auf einen vorgegeben pH-Wert im Bereich zwischen 5,3 und 5,9 (z. B. pH-Wert von ca. 5,5) unter anschließender Kontrolle des erreichten pH-Werts eingestellt. Anschließend werden hierzu die nachfolgenden in den Rezepturbeispielen spezifizierten Mengen der weiteren Wirk- und Inhaltsstoffe (1,8-Cineol, Dexpanthenol und Konservierungs-/Desinfektionsmittel (Antiseptikum) sowie gegebenenfalls vorhandene weitere Inhaltsstoffe, wie z. B. Emulgator, Verdickungsmittel etc.) hinzugegeben, und das Ganze wird dann durch gründliches Rühren klar gelöst. Nachfolgend wird das Ganze mit weiterem Wasser zum Endgewicht von 1.000 g aufgefüllt und homogen gerührt. Gegebenenfalls wird die Lösung über neutrale Cellulosefilter filtriert. Abschließend wird der pH-Wert noch einmal kontrolliert. Auch die übrigen entsprechenden Spezifikationen der Lösung werden auf die eingestellten bzw. vorgewählten Wertebereiche hin überprüft (z. B. Osmolalität; relative Dichte; mikrobiologische Reinheit und Sterilität; Ausschluss von Verunreinigungen, insbesondere Abbauprodukten der Inhalts- und Wirkstoffe; Viskosität; Aussehen; Brechungsindex). Ein Teil der erhaltenen Lösung wird schließlich in Enghalsflaschen aus Braunglas zu 10 ml oder 20 ml abgefüllt, welche wahlweise mit einer Tropfpipette oder einer Sprühdosierpumpe ausgerüstet werden können; zur bestimmungsgemäßen Anwendung können mehrmals täglich ein bis drei Tropfen bzw. ein bis zwei Sprühstöße in jedes Nasenloch gegeben und aufgezogen werden. Ein anderer Teil der erhaltenen Lösung wird für Stabilitätsuntersuchungen verwendet.

Nach dieser allgemeinen Herstellvorschrift werden die nachfolgend spezifizierten Rezepturen hergestellt. Die Rezepturen A4, A5, A6, A7, A13 und A14 sind nicht erfindungsgemäss.

**Rezeptur A1 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat/Sorbinsäure 1 : 1) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A2 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat/Sorbinsäure 5 : 1) | 0,20 | Ph. Eur. |
| Emulgator | 1,670 | Ph. Eur. |
| (Macroqolglycerolricinoleat) | | |
| Gereinigtes Wasser | 91,250 | Ph. Eur. |

**Rezeptur A3 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat/Sorbinsäure 1 : 5) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A4 (Angabe pro 1.000 g Zusammensetzung)**

| Inhaltsstoff | Menge / Gew.-% | Qualität |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydroqenphosphat (Dodekahydrat) | 0,027 | |
| Konservierungs-/Desinfektionsmittel (Antiseptikum) (Kaliumsorbat/Sorbinsäure 10:1) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A5 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat/Sorbinsäure 1 : 10) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A6 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A7 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydroqenphosphat (Dodekahydrat) | 0,027 | |
| Konservierungs-/Desinfektionsmittel (Antiseptikum) (Polyhexanid) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A8 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat/Sorbinsäure 1 : 1) | 0,20 | Ph. Eur. |
| Emulgator | 1,670 | Ph. Eur. |
| (Undecylenamidopropylbetain) | | |
| Gereinigtes Wasser | 91,250 | Ph. Eur. |

**Rezeptur A9 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydroqenphosphat (Dodekahydrat) | 0,027 | |
| Konservierungs-/Desinfektionsmittel (Antiseptikum) (Kaliumsorbat/Sorbinsäure 1 : 1) | 0,20 | Ph. Eur. |
| Emulgator | 1,670 | Ph. Eur. |
| (Poloxamer) | | |
| Gereinigtes Wasser | 91,250 | Ph. Eur. |

**Rezeptur A10 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydroqenphosphat (Dodekahydrat) | 0,027 | |
| Konservierungs-/Desinfektionsmittel (Antiseptikum) (Kaliumsorbat/Sorbinsäure 1 : 1) | 0,20 | Ph. Eur. |
| Emulgator | 1,670 | Ph. Eur. |
| (Cocoamidopropylbetain) | | |
| Gereinigtes Wasser | 91,250 | Ph. Eur. |

**Rezeptur A11 (Angabe pro 1.000 g Zusammensetzung)**

| **Inhaltsstoff** | **Menge / Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Citronensäure/Citrat-Puffersystem | 0,880 | |
| Konservierungs-/Desinfektionsmittel (Antiseptikum) (Kaliumsorbat/Sorbinsäure 1 : 1) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A12 (Angabe pro 1.000 g Zusammensetzung)**

| **Inhaltsstoff** | **Menge** / **Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kohlensäure/Bicarbonat-Puffersystem | 0,880 | |
| Konservierungs-/Desinfektionsmittel (Antiseptikum) (Kaliumsorbat/Sorbinsäure 1 : 1) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A13 (Angabe pro 1.000 g Zusammensetzung)**

| Inhaltsstoff | Menge / Gew.-% | Qualität |
|---|---|---|
| Cineol | 1,000 | Ph. Eur. |
| Dexpanthenol | 5,000 | Ph. Eur. |
| Puffersystem : | | Ph. Eur. |
| Citronensäure/Citrat-Puffersystem | 0,880 | |
| Konservierungs-/Desinfektionsmittel (Antiseptikum) (Kaliumsorbat) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

**Rezeptur A14 (Angabe pro 1.000 g Zusammensetzung)**

| **Inhaltsstoff** | **Menge** / **Gew.-%** | **Qualität** |
|---|---|---|
| Cineol | 1,000 | Ph. Eur. |
| Dexpanthenol | 5,000 | Ph. Eur. |
| Puffersystem: | | Ph. Eur. |
| Kohlensäure/Bicarbonat-Puffersystem | 0,880 | |
| Konservierungs-/Desinfektionsmittel (Antiseptikum) (Sorbinsäure) | 0,20 | Ph. Eur. |
| Emulgator | 1,670 | Ph. Eur. |
| (Macrogolglycerolricinoleat) | | |
| Gereinigtes Wasser | 91,250 | Ph. Eur. |

Erfindungsgemäss sind die Rezepturen A1-A3, A8-A12.

### Allgemeines Stabilitätsverhalten

Das Stabilitätsverhalten dieser Zusammensetzungen wird unter definierten Bedingungen untersucht (Lagerung jeweils bei 25 °C, bei einem Druck von 1.013,25 mbar [Atmosphärendruck] und bei einer relativen Luftfeuchtigkeit von 75 %). Die nachfolgenden Angaben beziehen sich auf die maximale Lagerfähigkeit (Anforderung an die Stabilität: Abbau Cineol ≤ 1 %, bezogen auf Cineol Abbau Dexpanthenol ≤ 1 % bezogen auf Dexpanthenol):

| **Zusammensetzung** | **Lagerfähigkeit** |
|---|---|
| A1 | 47,5 Monate |
| A2 | 45,5 Monate |
| A3 | 44,5 Monate |
| A4 | 41,5 Monate |
| A5 | 39,5 Monate |
| A6 | 35,5 Monate |
| A7 | 34,5 Monate |
| A8 | 36,5 Monate |
| A9 | 36,0 Monate |
| A10 | 36,5 Monate |
| A11 | 24,5 Monate |
| A12 | 21,5 Monate |
| A13 | 36,0 Monate |
| A14 | 36,5 Monate |

Wie die Untersuchungen zeigen, werden besonders gute Lagerfähigkeiten erhalten, wenn den erfindungsgemäßen Zusammensetzungen neben den eigentlichen Wirkstoffen (Cineol und Dexpanthenol) weitere Inhaltsstoffe auf Basis von Emulgatoren, Konservierungs-/Desinfektionsmitteln und/oder Puffersalzen zugesetzt werden. Besonders gute Ergebnisse werden erhalten, wenn ein Phosphatpuffersystem ("Kaliumdihydrogenphosphat/Natriummonohydrogenphosphatdodekahydrat") zum Einsatz kommt, als Emulgator ein Macrogolglycerolricinoleat verwendet wird und ein Konservierungs-/Desinfektionsmittel (Antiseptikum) auf Basis einer Sorbat/Sorbinsäure-Kombination, bevorzugt mit einem Sorbat/Sorbinsäure-Gewichtsverhältnis von etwa 1 : 1, eingesetzt wird. Überraschenderweise bewirkt eine Sorbat/Sorbinsäure-Kombination gegenüber den Einzelstoffen allein (d. h. entweder Sorbat allein oder aber Sorbinsäure allein) eine signifikante Steigerung der Stabilität (vgl. Rezepturen A13 und A14 im Vergleich zu Rezepturen A1 bis A5 und A8 bis A12), d. h. Sorbat allein oder aber Sorbinsäure allein zeigen im Rahmen der obigen Rezepturen jeweils nur ein in etwa gleiches Stabilisierungsverhalten wie Benzalkoniumchlorid oder Polyhexanid, wohingegen eine Sorbat/Sorbinsäure-Kombination überraschend eine synergistisch gesteigerte Stabilisierung bewirkt (wobei die Stabilisierungswirkung besonders gut ist, wenn das Sorbat/Sorbinsäure-Gewichtsverhältnis im Bereich von 9 : 1 bis 1 : 9, insbesondere 8 : 2 bis 2 : 8, vorzugsweise 7 : 3 bis 3 : 7, besonders bevorzugt 6 : 4 bis 4 : 6, ganz besonders bevorzugt 5,5 : 4,5 bis 4,5 : 5,5, liegt und noch mehr bevorzugt etwa 1 : 1 beträgt).

### Stabilitätsverhalten in Abhängigkeit vom pH-Wert

Entsprechend der vorstehenden allgemeinen Herstellvorschrift werden wässrige Zusammensetzungen nach der vorliegenden Erfindung jeweils mit 1,000 Gew.-% 1,8-Cineol und 5,000 Gew.-% Dexpanthenol (jeweils bezogen auf die Zusammensetzung) hergestellt.

Mit unterschiedlichen Mengen an Puffer und erforderlichenfalls unterschiedlichen Puffersystemen wird jeweils der pH-Wert der Zusammensetzungen variiert und das Stabilitätsverhalten untersucht (Lagerung jeweils bei 25 °C, bei einem Druck von 1.013,25 mbar [Atmosphärendruck] und bei einer relativen Luftfeuchtigkeit von 75 %).

Die nachfolgenden Angaben beziehen sich auf die maximale Lagerfähigkeit bei den unterschiedlichen angegeben pH-Werten (Anforderung an die Stabilität: Abbau Cineol ≤ 1 %, bezogen auf Cineol; Abbau Dexpanthenol ≤ 1 %, bezogen auf Dexpanthenol):

| **pH-Wert der Zusammensetzung** | **Lagerfähigkeit** |
|---|---|
| 3,0 | 27,5 Monate |
| 4,0 | 28,5 Monate |
| 4,5 | 30,5 Monate |
| 5,0 | 35,5 Monate |
| 5,3 | > 40 Monate |
| 5,5 | > 40 Monate |
| 5,9 | > 40 Monate |
| 6,5 | 32,5 Monate |
| 7,5 | 30,5 Monate |
| 8,0 | 27,5 Monate |
| 9,0 | 24,0 Monate |

Wie die Untersuchungen zeigen, wird die beste Lagerfähigkeit in einem pH-Wertebereich von 5,0 bis 6,5, insbesondere 5,3 bis 5,9, erreicht. Bei diesen Zusammensetzungen wird ein wie zuvor spezifiziertes pH-Wertpuffersystem auf Phosphatbasis (d. h. Dihydrogenphosphat/Monohydrogenphosphat-Puffersystem bzw. H₂PO₄⁻HPO₄²⁻-Puffer) eingesetzt.

### Untersuchungen zum antibakteriellen und antiviralen Wirkverhalten

Die erfindungsgemäßen Zusammensetzungen A1 bis A3 und die nicht erfindungsgemässen Zusammensetzungen A4 bis A7 werden auf ihr antibakterielles und antivirales Wirkverhalten untersucht. Eine weitere, nichterfindungsgemäße Zusammensetzung A13 wird untersucht, wobei die nichterfindungsgemäße Zusammensetzung A13 der erfindungsgemäßen Zusammensetzung A1 entspricht, wobei jedoch das Cineol durch einen entsprechenden Wasseranteil ersetzt ist.

Die antibakterielle Aktivität der Zusammensetzungen wird in einem Plattendiffusionstest sowie in einer Verdünnungstestreihe an 18 Bakterienstämmen (hierunter *Staphylococcus aureus, Haemophilus influenzae*, *Moraxella catarrhalis, Streptococcus pneumoniae, Bacillus sp., Citrobacter sp., Escherichia coli*, *Klebsiella sp.*, *Salmonella sp.* und *Vibrio cholera*) untersucht (Ermittlung der MIC-Konzentrationen [*Mininimal Inhibitory Concentrations* bzw. minimale Hemmkonzentrationen]). Die Zusammensetzungen A1 bis A7 zeigen eine gute antibakterielle Aktivität, wobei die Zusammensetzung A1 die beste antibakterielle Aktivität zeigt, gefolgt von den Zusammensetzungen A2, A3, A4, A5, A6 und A7 (in der Reihenfolge der antibakteriellen Wirksamkeit); die nichterfindungsgemäße Zusammensetzung A13 zeigt im Wesentlichen keine antibakterielle Aktivität.

Die antivirale Aktivität der Zusammensetzungen wird gegen IBV (Infektiöses Bronchitis-Virus, IBV Gray Stamm) in einem sogenannten MTT-Test in Vero-E6 (*African green monkey kidney cells* [Affennierenzellen]) untersucht (Ermittlung der IC₅₀-Konzentrationen). Die Zusammensetzungen A1 bis A7 zeigen eine gute antivirale Aktivität (Anti-IBV-Eigenschaften), wobei die Zusammensetzung A1 die beste antivirale Aktivität zeigt, gefolgt von den Zusammensetzungen A2, A3, A4, A5, A6 und A7 (in der Reihenfolge der antiviralen Wirksamkeit); die nichterfindungsgemäße Zusammensetzung A13 zeigt im Wesentlichen keine antivirale Aktivität.

### 2. Therapeutische Anwendung bei allergischen Rhinitiden

Die beschriebenen erfindungsgemäßen Zusammensetzungen A1 bis A3 und die nicht erfindungsgemässen Zusammensetzungen A4 bis A7 sowie die zuvor beschriebene nichterfindungsgemäße Zusammensetzung A13 werden therapeutisch bei allergischen Rhinitiden angewendet.

An akuten allergischen Rhinitiden ("Heuschnupfen") Erkrankte werden hierzu während der Dauer ihrer Erkrankung 5 Tage lang mit den Zusammensetzungen A1 bis A7 und A13 behandelt, wobei die Zusammensetzungen A1 bis A7 und A13 mehrmals täglich als Spray topisch appliziert werden. Weitere an akuten allergischen Rhinitiden Erkrankte werden mit einer nichterfindungsgemäßen Zusammensetzung A14 therapiert, wobei die nichterfindungsgemäße Zusammensetzung A14 keine Wirkstoffe enthält (Placebo).

Es wird eine Bewertung der Nasenatmungsbehinderung, der Rhinorrhö (Fließschnupfen), der Rötung der Nasenschleimhaut, der Trockenheit der Nasenschleimhaut, der Borkenbildung, des Heilungsverlaufs und der Verträglichkeit vorgenommen.

Alle untersuchten Zusammensetzungen zeigen eine ausgezeichnete Verträglichkeit. Bezüglichkeit der Beschleunigung des Heilungsverlaufs liefert die Zusammensetzung A1 das beste Ergebnis, gefolgt von den Zusammensetzungen A2, A3, A4, A5, A6 und A7 (in der Reihenfolge der Wirksamkeit bzw. der Beschleunigung des Heilungsverlaufs); die nichterfindungsgemäße Zusammensetzung A13 und die Zusammensetzung A14 führen im Wesentlichen zu keiner Beschleunigung des Heilungsverlaufs.

Insgesamt zeigen alle untersuchten Zusammensetzungen A1 bis A7 eine ausgezeichnete Wirksamkeit bei der Behandlung allergischer Rhinitiden ("Heuschnupfen"). Bezüglichkeit der Wirksamkeit liefert die erfindungsgemäße Zusammensetzung A1 das beste Ergebnis, gefolgt von den erfindungsgemäßen Zusammensetzungen A2, A3, und den nicht erfindungsgemässen Zusammensetzungen A4, A5, A6 und A7 (in der Reihenfolge der Wirksamkeit). Die nichterfindungsgemäßen Zusammensetzungen A13 und A14 zeigen im Wesentlichen keine Wirksamkeit.

Weitergehende Untersuchungen und Studien bestätigen darüber hinaus einen Synergismus der Wirkungen der Wirkstoffe (d. h. Cineol und Dexpanthenol) der erfindungsgemäßen Zusammensetzungen, was bei der Behandlung von Rhinitiden zu einer deutlicheren bzw. signifikanteren Besserung führt, und zwar über das Ausmaß der Einzelwirkstoffe deutlich hinausgehend.

## Patentansprüche

1. Cineolhaltige Zusammensetzung für die topische Applikation für die Behandlung von Rhinitiden,
wobei die Zusammensetzung in Kombination und jeweils in wirksamen Mengen
(a) Cineol, wobei das Cineol als Reinstoff vorliegt und frei von anderen Terpenen ist;
(b) Pantothenol oder dessen physiologisch unbedenkliche Ester und/oder Salze; und
(c) mindestens ein Konservierungsmittel und/oder Desinfektionsmittel (Antiseptikum) in relativen Mengen im Bereich von 0,05 bis 2 Gew.-%, bezogen auf die Zusammensetzung, wobei das Konservierungsmittel und/oder Desinfektionsmittel (Antiseptikum) eine Kombination von Sorbinsäure (Hexa-2,4-diensäure) einerseits und Sorbinsäuresalz (Sorbat) andererseits ("Sorbinsäure/Sorbat-Kombination") umfasst oder hieraus besteht,
enthält,
wobei die Komponente (c) die Kombination von Sorbinsäure einerseits und Sorbat andererseits mit einem Sorbinsäure/Sorbat-Gewichtsverhältnis im Bereich von 9 : 1 bis 1 : 9 umfasst.

2. Zusammensetzung nach Anspruch 1, wobei die Zusammensetzung die Komponente (a), bezogen auf die Zusammensetzung, in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,5 bis 1,8 Gew.-%, am meisten bevorzugt 0,7 bis 1,5 Gew.-%, enthält.

3. Zusammensetzung nach Anspruch 1 oder 2,
wobei die Zusammensetzung als Komponente (b) Pantothenol in Form von Dexpanthenol (D-Pantothenol) enthält; und/oder
wobei die Zusammensetzung die Komponente (b), bezogen auf die Zusammensetzung, in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%, enthält.

4. Zusammensetzung nach einem der vorangehenden Ansprüche,
wobei die Zusammensetzung die Komponente (c), bezogen auf die Zusammensetzung, in relativen Mengen im Bereich von 0,05 bis 1 Gew.-%, noch mehr bevorzugt 0,08 bis 0,8 Gew.-%, am meisten bevorzugt 0,1 bis 0,5 Gew.-%, enthält; und/oder
wobei die Komponente (c) die Kombination von Sorbinsäure einerseits und Sorbat andererseits mit einem Sorbinsäure/Sorbat-Gewichtsverhältnis im Bereich von 8 : 2 bis 2 : 8, vorzugsweise im Bereich von 7 : 3 bis 3 : 7, besonders bevorzugt im Bereich von 6 : 4 bis 4 : 6, ganz besonders bevorzugt im Bereich von 5,5 : 4,5 bis 4,5 : 5,5, noch mehr bevorzugt mit einem Sorbinsäure/Sorbat-Gewichtsverhältnis von etwa 1 : 1, umfasst.

5. Zusammensetzung nach einem der vorangehenden Ansprüche, wobei die Zusammensetzung außerdem als Komponente (d) mindestens einen Emulgator enthält.

6. Zusammensetzung nach einem der vorangehenden Ansprüche,
wobei die Zusammensetzung außerdem als Komponente (e) mindestens ein chemisches Puffersystem, insbesondere in Form von Puffersalz(en), enthält;
insbesondere wobei die Komponente (e) und/oder das chemische Puffersystem als ein Dihydrogenphosphat/Monohydrogenphosphat-Puffersystem ("H₂PO₄⁻/HPO₄²⁻-Puffer(system)" bzw. "Phosphatpuffer(system)"), insbesondere als ein Alkalidihydrogenphosphat/Alkalimonohydrogenphosphat-Puffersystem, vorliegt, bevorzugt mit einem Dihydrogenphosphat/Monohydrogenphosphat-Gewichtsverhältnis von größer 5:1, insbesondere im Bereich von 5:1 bis 200 : 1, insbesondere im Bereich von 6:1 bis 150 : 1, vorzugsweise im Bereich von 7:1 bis 100 : 1, besonders bevorzugt im Bereich von 10 : 1 bis 50 : 1.

7. Zusammensetzung nach einem der vorangehenden Ansprüche,
wobei die Zusammensetzung einen pH-Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, aufweist.

8. Zusammensetzung nach einem der vorangehenden Ansprüche,
wobei die Zusammensetzung außerdem als Komponente (f) mindestens ein weiteres Konservierungsmittel und/oder Desinfektionsmittel (Antiseptikum) enthält; und/oder
wobei die Zusammensetzung außerdem als Komponente (g) mindestens ein Verdickungsmittel enthält; und/oder
wobei die Zusammensetzung außerdem als Komponente (j) mindestens ein vorzugsweise imidazolinbasiertes alpha-Sympathomimetikum oder dessen physiologisch unbedenkliche Salze enthält.

9. Zusammensetzung nach einem der vorangehenden Ansprüche,
wobei die Zusammensetzung als wässrige Zusammensetzung vorliegt und/oder wobei die Zusammensetzung wässrig basiert ist und/oder wässrig formuliert vorliegt, insbesondere in Form einer wässrigen Lösung oder wässrigen Solubilisierung; und/oder
wobei die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist.

10. Zusammensetzung nach einem der vorangehenden Ansprüche,
wobei die Zusammensetzung bei einer Temperatur von 20 °C in Abwesenheit eines Verdickungsmittels eine dynamische Viskosität im Bereich von 1,001 bis 2 mPas, insbesondere 1,01 bis 1,9 mPas, vorzugsweise 1,05 bis 1,8 mPas, besonders bevorzugt 1,1 bis 1,5 mPas, aufweist (insbesondere bestimmt gemäß der Methode nach Ph. Eur. 7. Ausgabe, Grundwerk 2011, Abschnitt 2.2.9 Kapillarviskosimeter); oder aber
wobei die Zusammensetzung bei einer Temperatur von 20 °C in Anwesenheit eines Verdickungsmittels eine dynamische Viskosität von mindestens 1,5 mPas, insbesondere im Bereich von 1,5 bis 10⁵ mPas, vorzugsweise 1,6 bis 10⁴ mPas, besonders bevorzugt 1,7 bis 10³ mPas, noch mehr bevorzugt 2 bis 100 mPas, aufweist (insbesondere bestimmt gemäß der Methode nach Ph. Eur. 7. Ausgabe, Grundwerk 2011, Abschnitt 2.2.9 Kapillarviskosimeter).

11. Zusammensetzung nach einem der vorangehenden Ansprüche, wobei die Zusammensetzung mindestens einen weiteren Inhaltsstoff, insbesondere Hilfsstoff und/oder Additiv, aufweist, insbesondere ausgewählt aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

12. Zusammensetzung nach einem der vorangehenden Ansprüche zur Verwendung bei der prophylaktischen und/oder kurativen topischen Behandlung von Rhinitiden, insbesondere von *Rhinitis acuta* oder *Rhinitis allergica.*

13. Applikationsvorrichtung für die intranasale Applikation in Form eines Behältnisses mit Tropf- oder Sprüheinrichtung, enthaltend eine Zusammensetzung nach einem der vorangehenden Ansprüche.

14. Verfahren zur Stabilisierung einer cineolhaltigen Zusammensetzung für die topische Applikation für die Behandlung von Rhinitiden,
wobei die Zusammensetzung in Kombination und jeweils in pharmazeutisch wirksamen Mengen
(a) Cineol, wobei das Cineol als Reinstoff vorliegt und frei von anderen Terpenen ist;
(b) Pantothenol oder dessen physiologisch unbedenkliche Ester und/oder Salze;
enthält,
**dadurch gekennzeichnet,**
**dass** der cineolhaltigen Zusammensetzung als Komponente (c) **in relativen Mengen im** Bereich von 0,05 bis 2 Gew.-%, bezogen auf die Zusammensetzung mindestens ein Konservierungsmittel und/oder Desinfektionsmittel (Antiseptikum) zugesetzt wird, wobei das Konservierungsmittel und/oder Desinfektionsmittel (Antiseptikum) eine Kombination von Sorbinsäure (Hexa-2,4-diensäure) einerseits und Sorbinsäuresalz (Sorbat) andererseits ("Sorbinsäure/Sorbat-Kombination") umfasst oder hieraus besteht und wobei die Komponente (c) die Kombination von Sorbinsäure einerseits und Sorbat andererseits mit einem Sorbinsäure/Sorbat-Gewichtsverhältnis im Bereich von 9 : 1 bis 1 : 9 umfasst.

## Claims

1. Cineole-containing composition for topical application for the treatment of episodes of rhinitis,
the composition containing a combination of respectively effective amounts of
(a) cineole, wherein the cineole exists as the pure substance and is free of other terpenes;
(b) pantothenol or its physiologically harmless esters and/or salts;
and
(c) at least one preservative and/or disinfectant (antiseptic) in relative amounts in the range of 0.05 to 2% by weight, relative to the composition, wherein the preservative and/or disinfectant (antiseptic) comprises or consists of a combination of sorbic acid (2,4-hexadienoic acid) on the one hand and sorbic acid salt (sorbate) on the other hand ("sorbic acid/sorbate combination"),
wherein the component (c) comprises the combination of sorbic acid on the one hand and sorbate on the other hand with a sorbic acid/sorbate weight ratio in the range of 9:1 to 1:9.

2. The composition according to claim 1, wherein the composition contains component (a), relative to the composition, in relative amounts in the range of 0.001 to 10% by weight, particularly 0.002 to 5% by weight, preferably 0.01 to 3% by weight, preferably 0.05 to 3% by weight, particularly preferably 0.1 to 2.5% by weight, very particularly preferably 0.2 to 2% by weight, even more preferably 0.5 to 1.8% by weight, most preferably 0.7 to 1.5% by weight.

3. The composition according to claim 1 or 2,
wherein the composition contains as component (b) pantothenol in the form of dexpanthenol (D-pantothenol); and/or
wherein the composition contains component (b), relative to the composition, in relative amounts in the range of 0.01 to 10% by weight, in particular 0.1 to 8% by weight, preferably 0.5 to 7% by weight, preferably 1 to 6.5%, particularly preferably 2 to 6%, very particularly preferably 4 to 6%, even more preferably 4.5 to 5.5%.

4. The composition according to any of the preceding claims,
wherein the composition contains component (c), relative to the composition, in relative amounts in the range of 0.05 to 1% by weight, even more preferably from 0.08 to 0.8% by weight, most preferably 0.1 to 0.5% by weight; and/or
wherein component (c) comprises the combination of sorbic acid on the one hand and sorbate on the other hand with a sorbic acid/sorbate weight ratio in the range of 8:2 to 2:8, preferably in the range of 7:3 to 3:7, particularly preferably in the range of 6:4 to 4:6, very particularly preferably in the range of 5.5:4.5 to 4.5:5.5, even more preferably with a sorbic acid/sorbate weight ratio of about 1:1.

5. The composition according to any of the preceding claims, wherein the composition further contains as component (d) at least one emulsifier.

6. The composition according to any of the preceding claims,
wherein the composition also contains as component (e) at least one chemical buffer system, particularly in the form of buffering salt(s);
particularly wherein component (e) and/or the chemical buffer system exists as a dihydrogen phosphate/monohydrogen phosphate buffer system ("H₂PO₄⁻/HPO₄²⁻ buffer (system)" or "phosphate buffer (system)"), particularly as an alkali dihydrogen phosphate/alkali monohydrogen phosphate buffer system, preferably with a dihydrogen phosphate/monohydrogen phosphate weight ratio greater than 5:1, particularly in the range of 5:1 to 200:1, particularly in the range of 6:1 1 to 150:1 preferably in the range of 7:1 to 100:1, particularly preferably in the range of 10:1 to 50:1.

7. The composition according to any of the preceding claims,
wherein the composition has a pH in the range of 4.5 to 8.0, particularly in the range of 5.0 to 6.5, preferably in the range of 5.0 to 6.2, preferably in the range of 5.0 to 6.0, even more preferably in the range of 5.1 to 6.0, very particularly preferably in the range of 5.2 to 5.9, most preferably in the range of 5.3 to 5.9.

8. The composition according to any of the preceding claims,
wherein the composition also contains as component (f) at least one further preservative and/or disinfectant (antiseptic); and/or
wherein the composition also contains as component (g) at least one thickener; and/or wherein the composition also contains as component (j) at least one preferably imidazoline-based alpha-sympathomimetic drug or its physiologically harmless salts.

9. The composition according to any of the preceding claims,
wherein the composition exists as an aqueous composition and/or wherein the composition has an aqueous base and/or aqueous formulation, particularly in the form of an aqueous solution or aqueous solubilization; and/or
wherein the composition has an osmolality in the range of 300 to 600 mOsm/kg, particularly in the range of 310 to 550 mOsm/kg, preferably in the range of 300 to 525 mOsm/kg, preferably in the range of 325 to 510 mOsm/kg, particularly preferably in the range of 350 to 500 mOsm/kg.

10. The composition according to any of the preceding claims,
wherein the composition, at a temperature of 20 °C, in the absence of a thickening agent, has a dynamic viscosity in the range of 1.001 to 2 mPa·s, particularly 1.01 to 1.9 mPa·s, preferably 1.05 to 1.8 mPa·s, particularly preferably 1.1 to 1.5 mPa·s (in particular, determined by the method according to the Ph. Eur., 7th edition, basic legislation 2011, section 2.2.9 Capillary viscometer); or else
wherein the composition, at a temperature of 20 °C, in the presence of a thickening agent, has a dynamic viscosity of at least 1.5 mPa·s, particularly in the range of 1.5 to 10⁵ mPa·s, preferably 1.6 to 10⁴ mPa·s, particularly preferably 1.7 to 10³ mPa·s, even more preferably 2 to 100 mPa·s, (in particular, determined by the method according to the Ph. Eur., 7th edition, basic legislation 2011, section 2.2.9 Capillary viscometer).

11. The composition according to any of the preceding claims, wherein the composition comprises at least one further ingredient, particularly an adjuvant and/or additive, particularly selected from the group of processing aids, stabilizers, emulsifiers, antioxidants, humectants, thickeners, antiseptics, dyes, flavourings, fragrances, perfumes, extenders, binders, wetting agents, vitamins, trace elements, minerals, micronutrients and/or essential oils and combinations thereof.

12. The composition according to any of the preceding claims for use in the prophylactic and/or curative topical treatment of rhinitis episodes, especially *acute rhinitis* or *allergic rhinitis.*

13. An application device for intranasal administration in the form of a container with dropper or spraying device, containing a composition according to any of the preceding claims.

14. A method for stabilizing a cineole-containing composition for topical application for the treatment of rhinitis episodes,
wherein the composition contains a combination of respectively pharmaceutically effective amounts of
(a) cineole, wherein the cineole exists as the pure substance and is free of other terpenes;
(b) pantothenol or its physiologically harmless esters and/or salts;
**characterised in that**
at least one preservative and/or disinfectant (antiseptic) is added to the cineole-containing composition as component (c) **in relative amounts in the** range of 0.05 to 2% by weight, relative to the composition, wherein the preservative and/or disinfectant (antiseptic) comprises or consists of a combination of sorbic acid (2,4-hexadienoic acid) on the one hand and sorbic acid salt (sorbate) on the other hand ("sorbic acid/sorbate combination") and wherein component (c) comprises the combination of sorbic acid on the one hand and sorbate on the other hand with a sorbic acid/sorbate weight ratio in the range of 9:1 to 1:9.

## Revendications

1. Composition contenant du cinéol pour application topique dans le traitement des rhinites,
la composition en association et pour chacun en des quantités efficaces contenant
(a) du cinéol, le cinéol étant présent sous forme de substance pure et exempt d'autres terpènes ;
(b) du pantothénol ou ses esters et/ou sels physiologiquement acceptables ;
et
(c) au moins un conservateur et/ou un désinfectant (antiseptique) en des quantités relatives dans la plage comprise entre 0,05 et 2 % en poids, rapporté à la composition, le conservateur et/ou le désinfectant (antiseptique) comprenant une association d'acide sorbique (acide hexa-2,4-diénoïque) d'une part et de sel d'acide sorbique (sorbate) d'autre part (« association acide sorbique/sorbate ») ou en étant constituée,
le composant (c) comprenant l'association d'acide sorbique d'une part et de sorbate d'autre part, le rapport pondéral acide sorbique/sorbate se situant dans la plage comprise entre 9 : 1 à 1 : 9.

2. Composition selon la revendication 1, la composition contenant le composant (a), rapporté à la composition, en des quantités relatives dans la plage comprise entre 0,001 et 10 % en poids, en particulier entre 0,002 et 5 % en poids, de préférence entre 0,01 et 3 % en poids, de préférence entre 0,05 et 3 % en poids, de manière particulièrement préférée entre 0,1 et 2,5 % en poids, de manière tout particulièrement préférée entre 0,2 et 2 % en poids, de manière encore plus préférée entre 0,5 et 1,8 % en poids, idéalement entre 0,7 et 1,5 % en poids.

3. Composition selon la revendication 1 ou 2,
la composition contenant comme composant (b) du pantothénol sous la forme de dexpanthénol (D-pantothénol) ; et / ou
la composition contenant le composant (b), rapporté à la composition, en des quantités relatives dans la plage comprise entre 0,01 et 10 % en poids, en particulier entre 0,1 et 8 % en poids, de préférence entre 0,5 et 7 % en poids, de préférence entre 1 et 6,5 % en poids, de manière particulièrement préférée entre 2 et 6 % en poids, de manière encore plus préférée entre 4 et 6 % en poids, idéalement entre 4,5 et 5,5 % en poids.

4. Composition selon l'une quelconque des revendications précédentes,
la composition contenant le composant (c), rapporté à la composition, en des quantités relatives dans la plage comprise entre 0,05 et 1 % en poids, de manière particulièrement préférée entre 0,08 et 0,8 % en poids, le plus préférablement entre 0,1 et 0,5 % en poids ; et/ou
le composant (c) comprenant l'association d'acide sorbique d'une part et de sorbate d'autre part, le rapport pondéral acide sorbique/sorbate se situant dans la plage comprise entre 8 : 2 et 2 : 8, de préférence dans la plage comprise entre 7 : 3 et 3 : 7, de manière particulièrement préférée dans la plage comprise entre 6 : 4 et 4 : 6, mieux encore dans la plage comprise entre 5,5 : 4,5 et 4,5 : 5,5, idéalement, le rapport pondéral acide sorbique/sorbate étant d'environ 1 : 1.

5. Composition selon l'une quelconque des revendications précédentes, la composition contenant en outre, en tant que composant (d), au moins un émulsifiant.

6. Composition selon l'une quelconque des revendications précédentes,
la composition contenant en outre en tant que composant (e) au moins un système tampon chimique, notamment sous forme de sel(s) tampon(s) ;
en particulier le composant (e) et / ou le système tampon chimique étant utilisé en tant que système tampon dihydrogénophosphate/monohydrogénophosphate (« (système) tampon H₂PO₄⁻/HPO₄²⁻ » ou « (système) tampon phosphate »"), notamment en tant que système tampon hydrogénophosphate de métal alcalin/monohydrogénophosphate de métal alcalin, de préférence le rapport pondéral dihydrogénophosphate/monohydrogénophosphate étant supérieur à 5 : 1, en particulier dans la plage comprise entre 5 : 1 et 200 : 1, en particulier dans la plage comprise entre 6 : 1 et 150 : 1, de préférence dans la plage comprise entre 7 : 1 et 100 : 1, de manière particulièrement préférée dans la plage comprise entre 10 : 1 et 50 : 1.

7. Composition selon l'une quelconque des revendications précédentes,
la composition présentant un pH dans la plage comprise entre 4,5 et 8,0, en particulier dans la plage entre 5,0 et 6,5, de préférence dans la plage entre 5,0 et 6,2, de préférence dans la plage entre 5,0 et 6,0, plus préférablement dans la plage entre 5,1 et 6,0, mieux encore dans la plage entre 5,2 et 5,9, idéalement dans la plage entre 5,3 à 5,9.

8. Composition selon l'une quelconque des revendications précédentes,
la composition contenant en outre en tant que composant (f) au moins un autre conservateur et/ou désinfectant (antiseptique) ; et/ou
la composition contenant en outre en tant que composant (g) au moins un épaississant ; et/ou
la composition contenant en outre en tant que composant (j) au moins un alpha-sympathomimétique de préférence à base d'imidazoline ou ses sels physiologiquement acceptables.

9. Composition selon l'une quelconque des revendications précédentes,
la composition étant présente sous la forme d'une composition aqueuse et/ou la composition ayant une base aqueuse et/ou se présentant sous la forme d'une formulation aqueuse, en particulier sous la forme d'une solution aqueuse ou d'une solubilisation aqueuse ; et/ou
la composition ayant une osmolalité dans la plage comprise entre 300 et 600 mOsm/kg, en particulier dans la plage comprise entre 310 et 550 mOsm/kg, de préférence dans la plage comprise entre 300 et 525 mOsm/kg, de préférence dans la plage comprise entre 325 et 510 mOsm/kg, de préférence encore dans la plage comprise entre 350 et 500 mOsm/kg.

10. Composition selon l'une quelconque des revendications précédentes,
la composition à une température de 20° C en l'absence d'un épaississant présentant une viscosité dynamique dans la plage comprise entre 1,001 et 2 mPa·s, en particulier entre 1,01 et 1,9 mPa·s, de préférence entre 1,05 et 1,8 mPa·s, de préférence encore entre 1,1 et 1,5 mPa·s (notamment déterminée par la méthode selon Ph. Eur. 7ème édition, ouvrage de base 2011, section 2.2.9 viscosimètre capillaire) ; ou bien
la composition à une température de 20°C en présence d'un épaississant présentant une viscosité dynamique d'au moins 1,5 mPa·s, en particulier dans la plage comprise entre 1,5 et 10⁵ mPa·s, de préférence entre 1,6 et 10⁴ mPa·s, de préférence encore entre 1,7 et 10³ mPa·s, plus préférentiellement entre 2 et 100 mPa·s (en particulier déterminée par la méthode de Ph. Eur. 7ème édition, ouvrage de base 2011, section 2.2.9 viscosimètre capillaire).

11. Composition selon l'une des revendications précédentes, la composition présentant au moins un autre ingrédient, en particulier un adjuvant et/ou un additif, notamment choisi dans le groupe des auxiliaires technologiques, des stabilisants, des émulsifiants, des antioxydants, des humectants, des épaississants, des antiseptiques, des colorants, des arômes, des substances odoriférantes, des parfums, des diluants, des liants, des agents mouillants, des vitamines, des oligo-éléments, des minéraux, des micronutriments et/ou des huiles essentielles et de leurs combinaisons.

12. Composition selon l'une quelconque des revendications précédentes, destinée à être utilisée dans le traitement topique prophylactique et/ou curatif des rhinites, en particulier de la *rhinitis acuta* ou de la *rhinitis allergica.*

13. Dispositif d'administration pour l'administration intranasale sous la forme d'un contenant doté d'un compte-goutte ou d'un pulvérisateur, contenant une composition selon l'une des revendications précédentes.

14. Procédé de stabilisation d'une composition contenant du cinéol pour l'administration topique dans le traitement des rhinites,
la composition en association et pour chacun en des quantités pharmaceutiquement efficaces contenant
(a) du cinéol, le cinéol étant présent sous forme de substance pure et exempt d'autres terpènes ;
(b) du pantothénol ou ses esters et/ou sels physiologiquement acceptables ;
**caractérisé en ce qu'**
on ajoute à la composition contenant du cinéol en tant que composant (c) **en des quantités relatives** dans la plage comprise entre 0,05 et 2 % en poids, rapporté à la composition, au moins un conservateur et/ou un désinfectant (antiseptique), le conservateur et/ou le désinfectant (antiseptique) comprenant une association d'acide sorbique (acide hexa-2,4-diénoïque) d'une part et de sel d'acide sorbique (sorbate) d'autre part (« association acide sorbique/sorbate ») ou en étant constitué, le composant (c) comprenant l'association d'acide sorbique d'une part et de sorbate d'autre part, le rapport pondéral acide sorbique/sorbate étant dans la plage comprise entre 9 : 1 à 1 : 9.
